# EUROPÄISCHE PATENTSCHRIFT

(11) **EP 0 646 132 B1**
(45) Veröffentlichungstag und Bekanntmachung des Hinweises auf die Patenterteilung: **11.08.1999**
(21) Anmeldenummer: 94913578.4
(22) Anmeldetag: 12.04.1994
(51) Int. Cl.: C07K 14/47, A61K 38/16, A61K 39/00, C12P 21/00, C07H 13/04

(54) **AMINOZUCKER, GLYCOPROTEINE, VERFAHREN ZU IHRER HERSTELLUNG, SIE ENTHALTENDE ARZNEIMITTEL UND IHRE VERWENDUNG**
AMINO SUGARS, GLYCOPROTEINS, METHOD OF PREPARING THEM, MEDICINES CONTAINING THEM AND THEIR USE
GLYCOSYLAMINES, GLYCOPROTEINES, LEUR PROCEDE DE PREPARATION, MEDICAMENTS LES CONTENANT ET LEUR UTILISATION

(30) Priorität: 12.04.1993 DE 4311580
(43) Veröffentlichungstag der Anmeldung: 05.04.1995
(73) Patentinhaber: REUTTER, Werner, D-14195 Berlin (DE)
(72) Erfinder: REUTTER, Werner, D-14195 Berlin (DE); SCHÜLER, Cora, D-1071 Berlin (DE); KEPPLER, Oliver, D-6900 Heidelberg (DE); PAWLIKA, Michael, D-6925 Eschelbronn (DE); KAYSER, Holger, D-1000 Berlin 37 (DE)
(74) Vertreter: Hartmann, Günter, Dr. Dipl.-Chem.
(86) Internationale Anmeldenummer: EP9401126
(87) Internationale Veröffentlichungsnummer: WO9424167

(56) Entgegenhaltungen:
- JOURNAL OF BIOLOGICAL CHEMISTRY. Bd. 267, Nr. 24 , 25. August 1992 , BALTIMORE US Seiten 16934 - 16938 H KAYSER ET AL. 'Biosysnthesis of a nonphysiological sialic acid in different rat organs using N-Propanoyl-D-hexosamines as precursors'
- FEBS LETTERS. Bd. 301, Nr. 2 , April 1992 , AMSTERDAM NL Seiten 137 - 140 H KAYSER ET AL. 'Incorporation of N-acyl-2-amino-2-deoxy-hexoses into glycosphingolipids of the pheocromocytoma cells line PC 12'
- CARBOHYDRATE RESEARCH Bd. 96 , 1981 , AMSTERDAM NL Seiten 259 - 270 H-J GRÜNHOLZ ET AL. 'Inhibition of in vitro biosynthesis of N-acetylneuraminic acid by N-acyl- and N-alkyl-2-amino-2-deoxyhexoses'
- EXPERIENTIA Bd. 49, Nr. 10 , 15. Oktober 1993 , BASEL CH Seiten 885 - 887 H KAYSER ET AL. 'New amino sugars analogues are incorporated at different rates into glycoproteins of mouse organs'

## Beschreibung

Die Erfindung betrifft Glycoproteine der nachstehend angegebenen allgemeinen Formeln (I) und (I'), Verfahren zu ihrer Herstellung und sie enthaltende pharmazeutische Mittel zur Stimulierung des Wachstums und der Differenzierung von menschlichen und tierischen Zellen des Immunsystems und zur Verhinderung der Adhäsion von Leukozyten, Thrombozyten und Tumorzellen an Gefäßendothelzellen; zur Stimulierung des Immunsystems, insbesondere von T-Lymphozyten, zur Infektabwehr, zur Behandlung von Immunschwächen, Tumorerkrankungen einschließlich Metastasierungsprozessen, Infektionserkrankungen (Viren, Bakterien, Parasiten, Protozoen) und Kreislaufversagen, insbesondere Gefäßverschlüssen und Septikämien, bei Mensch und Tier; zur Hemmung der Bindung eines Liganden an seinen sialylierten Zelloberflächenrezeptor; zur Hemmung der Bindung eines mikrobiellen Pathogens (Virus, Bakterium, Parasit, Protozoon) oder Toxins an die Wirtszelle über einen sialylierten Rezeptor durch in vivo-Modulation von Neuraminsäuren; zur biosynthetischen Herstellung von Liganden oder Rezeptoren mit modifizierter Neuraminsäure und Verwendung derselben zur Kompetition physiologischer oder pathologischer Ligand-Rezeptor-Interaktionen; zur in vitro-Beeinflussung des Infektionsverlaufs humaner Immundefizienz-Viren (z.B. HIV-1 und HIV-2) sowie zur in vivo-Prävention der Infektion humaner Immundefizienz-Viren (z.B. HIV-1 und HIV-2); und zur Behandlung von parasitären Erkrankungen, insbesondere von Trypanosomiasen, Leishmaniasen, Trichomoniasis, Giardiasis, Amöbiasis, Malaria, Pneumozystose, Schistosomiasis (Bilharziose) und Echinokokkose.

Als Glycoproteine werden Proteine bezeichnet, die kovalent gebundene Kohlenhydrate enthalten. In tierischen Organismen kommen Glykoproteine als wesentliche Bestandteile von Zellmembranen sowie als lösliche Komponenten von Körper-Flüssigkeiten und der extrazellulären Matrix vor. Die Kohlenhydrate sind zu Ketten verknüpft (Oligosaccharide) und können auf unterschiedliche Weise mit dem Proteingerüst verknüpft sein. Sie enthalten als wichtige Bestandteile der Zellmembran Sialinsäure (Derivate der 2-Keto-3-desoxy-D-glycero-D-galacto-nonulopyranosidonsäure (KDN)), der eine bedeutende Rolle bei biologischen Prozessen zukommt.

Aufgrund ihres Bindungstyps werden die Oligosaccharide unterschiedlichen Gruppen zugeordnet. Die Oligosaccharide von Säugetierglycoproteinen sind am häufigsten N-glycosidisch an einen Asparaginrest der Polypeptidkette gebunden (N-Glycane). In dieser Gruppe finden sich einerseits sezernierte Glykoproteine mit unterschiedlichen Funktionen, z.B. lösliche Enzyme, Immunglobuline und Hormone, andererseits Membranglycoproteine, z.B. Membranenzyme, Transportproteine sowie Rezeptorproteine. Eine weitere Gruppe bilden die O-glycosidisch über einen Galactose-, N-Acetylgalactosamin- oder Xyloserest an einen Serin- oder Threoninrest der Polypeptidkette gebundenen Oligosaccharide (O-Glycane). Sie werden vor allem in Muzinen gefunden, welche die Schleimepithelien des Atem-, Urogenital- und Gastrointestinaltraktes sowie von Tumorzelloberflächen auskleiden. Zusammen mit N-Glycanen kommen sie jedoch auch in Immunglobulinen und anderen Glycoproteinen vor. Zu den O-Glycanen gehören auch die Oligosaccharide der Proteoglycane, die sich durch einen besonders hohen Kohlenhydratanteil auszeichnen. In diesen in der extrazellulären Matrix vorkommenden Glycokonjugaten können die Oligosaccharide über einen Galactose-, N-Acetylgalactosamin- oder Xyloserest an das Polypeptidgerüst gebunden sein.

Die Glycoproteine, bestehend aus Monosacchariden und Eiweiß, werden häufig mit den Glycolipiden als Glycokonjugate zusammengefaßt. Die Zuckerkomponenten der Glycoproteine, die mit wenigen Ausnahmen weniger als 50 % des Gesamt-Glycoproteins ausmachen, sind über durch Glycosidasen spaltbare O- oder N-glycosidische Bindungen mit dem Peptidanteil verknüpft. Als Kohlenhydrate finden sich in den Glycoproteinen Hexosen (Galactose, Mannose, seltener Glucose), N-Acetylhexosamine, N-Acylneuraminsäuren, Fucose und andere. Zur Identifizierung und Bestimmung der Glycoproteine eignet sich in erster Linie die Affinitätschromatographie mit pflanzlichen Lectinen als Liganden (z.B. Concanavalin A oder Weizenkeimagglutinin oder andere).

Zu den Glycoproteinen gehören fast alle Membranglycoproteine, Serumproteine, Plasmaproteine, die Blutgruppensubstanzen, viele Enzyme und Proteohormone, alle Antikörper, die Chalone, Muzine, Lectine, Bindine, Fibronectin, der Intrinsic-Faktor und dgl.

Als Membran- oder Oberflächenproteine spielen manche Glycoproteine für die Pathogenität von Viren eine Rolle. Hier wie bei anderen rezeptorspezifischen zellulären Wechselwirkungen sind die Kohlenhydrat-Komponenten für Erkennungsprozesse auf molekularer Ebene verantwortlich.

Über bestimmte Zuckerstrukturen auf Rezeptoren heften einige Bakterien und Viren an ihre Zielzellen an.

Besonders bedeutungsvoll sind Oligosaccharidstrukturen auch im Hinblick auf die Zell-Zell- bzw. Zell-Matrix-Interaktion. So vermitteln die Oligosaccharide von Glycoproteinen die Adhäsion von Neuronalzellen und die Bindung von Lymphozyten an spezifische Endothelzellen. Außerdem können Oligosaccharide als antigene Determinanten von Glycoproteinen dienen. Auch während der Embryogenese und der Organogenese sind Kohlenhydrat-Kohlenhydrat-Wechselwirkungen wesentlich an der spezifischen Zellerkennung beteiligt.

Die maligne Transformation von Zellen wird von charakteristischen Veränderungen der Oligosaccharidstrukturen von Glycoproteinen begleitet. Inwieweit veränderte Oligosaccharidstrukturen von Tumorzellen und Zellglycoproteinen Ursache oder Wirkung der Tumorentstehung und Metastasierung sind, ist bis jetzt nicht eindeutig geklärt.

In Krankheitsfällen, in denen das Immunsystem beteiligt ist, ist die Unterstützung des Immunsystems durch Verabreichung von Substanzen, die Zellen des Immunsystems stimulieren, erforderlich. Die Suche nach Wirkstoffen zur Stimulierung des Immunsystems ist daher ein vordringliches Ziel pharmakologischer Forschung. Wirkungsvolle Immunstimulantien mit möglichst wenig Nebenwirkungen sind aber bisher nicht bekannt.

Aufgabe der Erfindung war es daher, Substanzen zu finden, mit deren Hilfe es möglich ist, wirkungsvoll und spezifisch das Immunsystem zu stimulieren.

Es wurde nun überraschend gefunden, daß diese Aufgabe erfindungsgemäß gelöst werden kann mit neuen Glycoproteinen der nachstehend angegebenen allgemeinen Formeln (I) und (I') und neuen Aminozuckern (Neuraminsäure-Vorstufen-Analoga) der nachstehend angegebenen allgemeinen Formel (II). Sie haben eine proliferationsstimulierende Wirkung auf tierische und menschliche Zellen des Immunsystems.

Gegenstand der Erfindung sind gemäß einem ersten Aspekt neue Glycoproteine der allgemeinen Formel (I) 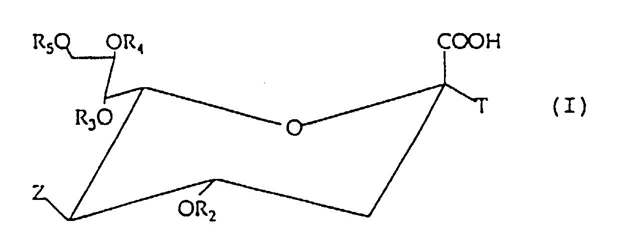 worin bedeuten:
- Z: -NHR, worin R den Pentanoyl-, Hexanoyl-, Heptanoyl- oder Crotonoylrest einschließlich seiner ein- oder mehrfach hydroxylierten Analoga darstellt
- R₁, R_{1'} , R_{1"} , R₂, R₃, R₄ und R₅,: die gleich oder verschieden sein können, jeweils Wasserstoff, einen linearen oder verzweigten Alkylrest mit 1 bis 20 Kohlenstoffatomen (CₙH₂ₙ₊₂, n = 1 bis 20), vorzugsweise 1 bis 7 Kohlenstoffatomen; einen linearen oder verzweigten Alkenylrest mit 3 bis 20 Kohlenstoffatomen (CₙH₂ₙ, n = 3 bis 20, Position der Doppelbindung an Cₙ n = 2 bis 19), vorzugsweise 3 bis 10 Kohlenstoffatomen; einen linearen oder verzweigten Alkinylrest mit 3 bis 20 Kohlenstoffatomen (CₙH₂ₙ₋₂, n = 3 bis 20, Position der Dreifachbindung an Cₙ n = 2 bis 19), vorzugsweise 3 bis 10 Kohlenstoffatomen; einen Alkenyl- bzw. Alkinylrest mit 2 oder mehr Doppelbindungen bzw. Dreifachbindungen mit 4 bis 20, vorzugsweise 7 bis 12 Kohlenstoffatomen; einen Arylrest mit 6 bis 20 Kohlenstoffatomen, vorzugsweise einen Phenylrest; einen linearen oder verzweigten, gesättigten oder ein- oder mehrfach ungesättigten Acylrest (-CO-R₁) mit insgesamt 1 bis 20, vorzugsweise 1 bis 7 Kohlenstoffatomen einschließlich seiner ein- oder mehrfach hydroxylierten Analoga; einen Aroylrest mit 6 bis 20, vorzugsweise 6 bis 10 Kohlenstoffatomen; einen Carbonylamidrest der Formel
-CONH₂, -CONHR₁, -CONR₁R_{1'} oder -CONR₁R_{1'}R_{1"} ; einen linearen oder verzweigten, gesättigten oder ein- oder mehrfach ungesättigten Thioacylrest (-CS-R₁) mit insgesamt 1 bis 20, vorzugsweise 1 bis 7 Kohlenstoffatomen; oder einen Thiocarbamidrest der Formel -CS-NH₂, -CS-NHR₁, -CS-NR₁R_{1'} oder -CS-NR₁R_{1'}R_{1"};
wobei jeder der vorgenannten Reste außer H gegebenenfalls ein- oder mehrfach substituiert sein kann durch Halogen, insbesondere Fluor, Chlor, Brom oder Jod, Hydroxy-, Epoxy-, Amino-, Mercaptan-, Phenyl-, Phenol- oder Benzylgruppen, und
- T: einen Mono-, Di- oder Oligosaccharidrest mit bis zu 40 glykosidisch miteinander verknüpften, gegebenenfalls verzweigten Zuckerresten, die Furanose- und/oder Pyranoseringe darstellen und 5 bis 230 Kohlenstoffatome enthalten und N- oder O-glycosidisch an Polypeptide gebunden sind, und

Glycoproteine der allgemeinen Formel (I') für einen Glycosylphosphatidylinosit(GPI)-Anker 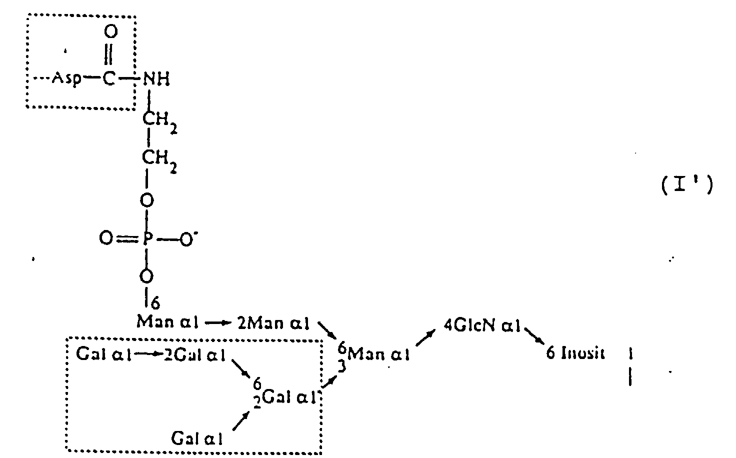 worin bedeuten:
- Gal =: Galactose,
- Man =: Mannose
- Asp =: Asparagin
- GlcN =: Glucosamin
wobei GlcN ersetzt sein kann durch ein Neuraminsäure-Vorstufen-Analogon der Formel (II)

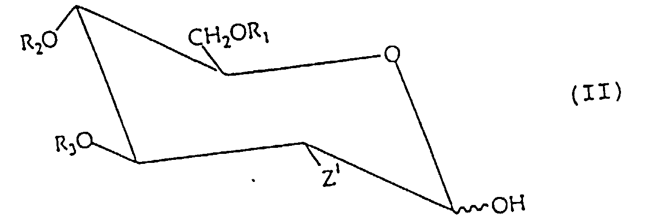

in der R₁, R₂ und R₃ die oben angegebenen Bedeutungen haben und Z' die gleichen Bedeutungen wie Z hat und neben der äquatorialen Position auch die axiale Position einnehmen kann und wobei außerdem bei äquatorialer Position von Z' der Rest -OR₂ in axialer Position stehen kann.

Besonders bevorzugte erfindungsgemäße Glycoproteine der vorstehend angegebenen allgemeinen Formel (I) sind solche, in denen T steht
für einen Saccharidrest mit N-Glycan-Struktur der Formel (Ia) 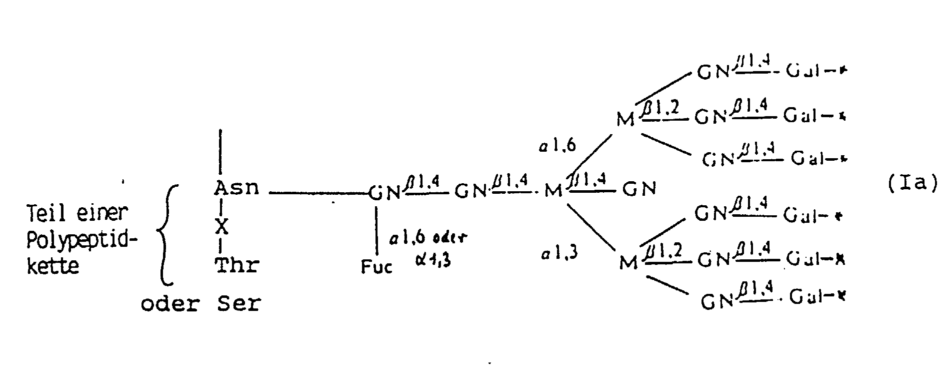 worin bedeuten:
- Gal =: Galactose,
- GN =: N-Acetyl-D-glucosamin,
- M =: D-Mannose,
- Fuc =: Fucose,
- Asn =: Asparagin,
- X =: eine beliebige Aminosäure außer Prolin,
- Thr =: Threonin,
- Ser =: Serin,
- * =: T-Verknüpfungsstelle (1 bis 6 Molekülreste)
wobei beide peripheren Reste M durch 1 bis 3 Trisaccharide substituiert sein können; oder
für einen Saccharidrest mit O-Glycan-Struktur der allgemeinen Formel (Ib): 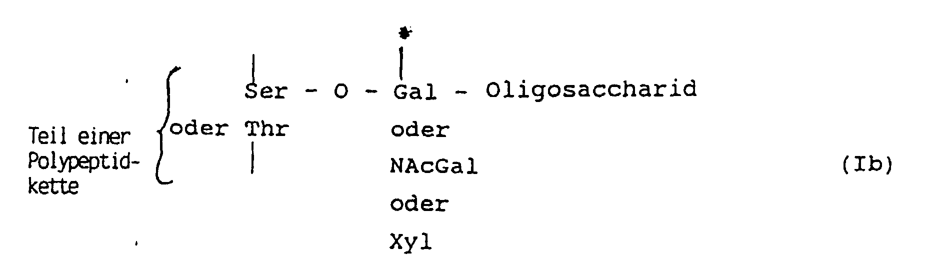 worin bedeuten:
- Gal =: Galactose
- Thr =: Threonin
- Ser =: Serin
- Xyl =: Xylose
- NAcGal =: N-Acetyl-galactosamin
- * =: T-Verknüpfungsstelle,
wobei in den obigen Formeln (Ia) und (Ib) die Galactose (Gal) ersetzt sein kann durch 2-Desoxy-galactose oder 2-Desoxy-2-halogenid(F, Cl, Br, J)-galactose.

Besonders bevorzugte Glycoproteine der Erfindung sind solche der obengenannten Formel (I), in denen dann, wenn T einen Saccharidrest mit N-Glycanstruktur oder einen Saccharidrest mit O-Glycanstruktur darstellt, GN steht für einen Rest der allgemeinen Formel (II): 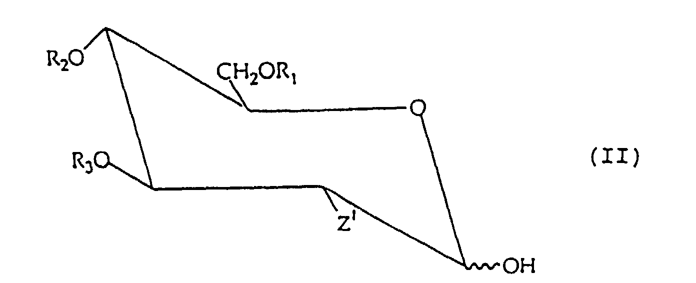 in der R₁, R₂ und R₃ die oben angegebenen Bedeutungen haben und Z' die gleichen Bedeutungen wie Z hat und neben der äquatorialen Position auch die axiale Position einnehmen kann, und wobei außerdem bei äquatorialer Position von Z' der Rest -OR₂ in axialer Position stehen kann.

Ganz besonders bevorzugte Glycoproteine der Erfindung sind auch solche, in denen R₂ bis R₅ für H oder CH₃ steht.

Gemäß einem weiteren Aspekt betrifft die vorliegende Erfindung Aminozucker ((Neuraminsäure-Vorstufen-Analoga) der allgemeinen Formel (II): 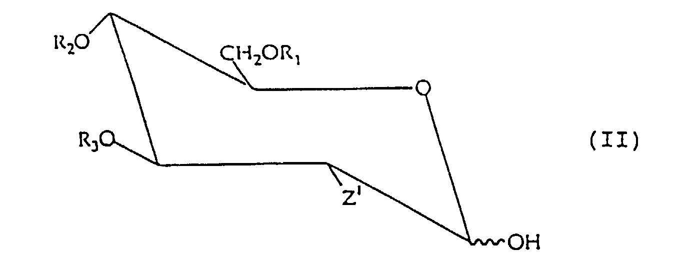 in der Z' und R₁, R₂ und R₃ die vorstehend angegebenen Bedeutungen haben.

Die erfindungsgemäßen neuen Aminozucker der allgemeinen Formel (II) und die daraus gebildeten neuen Glycoproteine der allgemeinen Formeln (I) und (I') stellen neue Substanzklassen dar, die als potente Wirkstoffe zur Behandlung von Krankheiten eingesetzt werden können, an denen Zellen der spezifischen und unspezifischen Immunabwehr, Tumorzellen, Leukozyten, Thrombozyten und Gefäßendothelzellen beteiligt sind. Die Verbindungen können gezielt über eine Modulation Membran-ständiger Rezeptoren wirken, die an der Regulation des Wachstums und der Differenzierung sowie der Adhäsion von Zellen des Immunsystems, von Tumoren und von Gefäßen beteiligt sind. Eine Immunstimulation ist notwendig beim Heilungsprozeß infektiöser und tumorbedingter Krankheiten. Weiterhin verhindern diese Verbindungen die Adhäsion von Leukozyten oder Tumorzellen auf Gefäßendothelzellen bei septischem Schock oder bei der Metastasierung. Die Verabreichung dieser Substanzen, insbesondere der Substanzen der Formel (II), führt zu keinen erkennbaren Nebenwirkungen.

Die erfindungsgemäßen Aminozucker (II) und Glycoproteine (I) und (I'), die neuartige Immunstimulantien darstellen, können als Stimulantien für Zellen des Immunsystems eingesetzt werden. Dadurch ist eine Stärkung des Immunsystems bei immungeschwächten Organismen möglich. Sie zeichnet sich durch hohe Zell-Spezifität und fehlende Nebenwirkungen aus.

Die in den Formeln (I), (I') und (II) genannten Reste werden nachstehend näher erläutert.

Beispiele für die obengenannten Alkylreste mit 1 bis 20, vorzugsweise 1 bis 7 Kohlenstoffatomen sind beispielsweise Propyl-, Butyl-, Pentyl-, Hexyl-, Isopropyl- und Pivalinylreste.

Beispiel für geeignete Alkenylreste sind Propenyl-, But-2-enyl-, But-3-enyl-, Pent-2-enyl- und Pent-3-enyl-Reste.

Beispiele für geeignete Alkenylreste mit mehreren Doppelbindungen sind Pent-2,4-dienyl- und Hex-2,4-dienyl-Reste.

Beispiele für geeignete Substituentengruppen sind Chlorethyl-, Dichlorethyl-, Trichlorethyl-, p-Chlorophenyl-, Hydroxymethyl-, 3-Hydroxypropyl- und p-Hydroxyphenylgruppen.

Die Sialinsäure ist Bestandteil des Glykoproteins (I) bzw. Aminozuckers (II).

Beispiele für langkettige Acylreste sind Caproyl-, Octoyl-, Lauroyl-, Myristoyl-, Palmitoyl-, Stearoyl-, Oleyl-, Linoleyl-, Linolenoyl- und Arachidonoyl-Reste sowie deren ein- oder mehrfach hydroxylierten Analoga.

Beispiele für geeignete Monosaccharid-, Disaccharid- und Oligosaccharid-Reste sind D-Glucosyl-, D-Galactosyl-, D-Mannosyl-, Xylosyl-, Inosyl-, Ribosyl-, Arabinosyl-, Fructosyl-, Sorbosyl-, Lactosyl-, Saccharosyl-, Trehalosyl-, Maltosyl-, Cellobiosyl- und höhere Saccharidreste, wie Raffinosyl-, Fucosyl-, Chitobiosyl-, Chitobiosemannosyl-, Rutinosyl- und Rhamnosyl-Reste. Es sind sowohl 1Â- als auch 1β-Verknüpfungen möglich. Ein oder mehrere Zuckerringe können auch als Zuckeramine wie Glucosamin, Mannosamin und Galactosamin vorliegen.

Gegenstand der Erfindung sind ferner Verfahren zur in vivo-Herstellung der Verbindungen der vorstehend angegebenen Formeln (I), (I') und (II) durch parenterale oder enterale Verabreichung einer 2-Desoxy-2-amino-mannose, -glucose oder -galactose, deren Aminogruppe durch R₁ substituiert ist, vorzugsweise von N-Propanoyl-, N-Butanoyl-, N-Pentanoyl-, N-Hexanoyl-, N-Heptanoyl- oder N-Crotonoyl-D-mannosamin, an Mensch oder Tier.

Gemäß einer weiteren Ausführungsform der Erfindung können die in vivo gebildeten Glycoproteine der allgemeinen Formeln (I) und (I') auf an sich bekannte Weise gewonnen (abgetrennt) werden für den therapeutischen Einsatz.

Gegenstand der Erfindung sind außerdem pharmazeutische Mittel, die dadurch gekennzeichnet sind, daß sie als Wirkstoff, gegebenenfalls in Kombination mit anderen Wirkstoffen sowie üblichen pharmazeutischen Trägern und/oder Hilfsstoffen, enthalten
mindestens ein Glycoprotein der Formel (I) 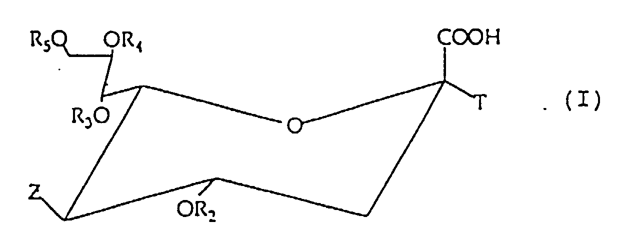 worin bedeuten:
- Z: -NHR, worin R den Pentanoyl-, Hexanoyl-, Heptanoyl- oder Crotonoylrest einschließlich seiner ein- oder mehrfach hydroxylierten Analoga darstellt
- R₁, R_{1'} , R_{1"} , R₂, R₃, R₄ und R₅,: die gleich oder verschieden sein können, jeweils Wasserstoff, einen linearen oder verzweigten Alkylrest mit 1 bis 20 Kohlenstoffatomen (CₙH₂ₙ₊₂, n = 1 bis 20), vorzugsweise 1 bis 7 Kohlenstoffatomen; einen linearen oder verzweigten Alkenylrest mit 3 bis 20 Kohlenstoffatomen (CₙH₂ₙ, n = 3 bis 20, Position der Doppelbindung an Cₙ n = 2 bis 19), vorzugsweise 3 bis 10 Kohlenstoffatomen; einen linearen oder verzweigten Alkinylrest mit 3 bis 20 Kohlenstoffatomen (CₙH₂ₙ₋₂, n = 3 bis 20, Position der Dreifachbindung an Cₙ n = 2 bis 19), vorzugsweise 3 bis 10 Kohlenstoffatomen; einen Alkenyl- bzw. Alkinylrest mit 2 oder mehr Doppelbindungen bzw. Dreifachbindungen mit 4 bis 20, vorzugsweise 7 bis 12 Kohlenstoffatomen; einen Arylrest mit 6 bis 20 Kohlenstoffatomen, vorzugsweise einen Phenylrest; einen linearen oder verzweigten, gesättigten oder ein- oder mehrfach ungesättigten Acylrest (-CO-R₁) mit insgesamt 1 bis 20, vorzugsweise 1 bis 7 Kohlenstoffatomen einschließlich seiner ein- oder mehrfach hydroxylierten Analoga; einen Aroylrest mit 6 bis 20, vorzugsweise 6 bis 10 Kohlenstoffatomen; einen Carbonylamidrest der Formel-CONH₂, -CONHR₁, -CONR₁R_{1'} oder -CONR₁R_{1'}R_{1"} ; einen linearen oder verzweigten, gesättigten oder ein- oder mehrfach ungesättigten Thioacylrest (-CS-R₁) mit insgesamt 1 bis 20, vorzugsweise 1 bis 7 Kohlenstoffatomen; oder einen Thiocarbamidrest der Formel -CS-NH₂, -CS-NHR₁, -CS-NR₁R_{1'} oder -CS-NR₁R_{1'}R_{1"};
wobei jeder der vorgenannten Reste außer H gegebenenfalls ein- oder mehrfach substituiert sein kann durch Halogen, insbesondere Fluor, Chlor, Brom oder Jod, Hydroxy-, Epoxy-, Amino-, Mercaptan-, Phenyl-, Phenol- oder Benzylgruppen, und
- T: einen Mono-, Di- oder Oligosaccharidrest mit bis zu 40 glykosidisch miteinander verknüpften, gegebenenfalls verzweigten Zuckerresten, die Furanose- und/oder Pyranoseringe darstellen und 5 bis 230 Kohlenstoffatome enthalten und N- oder O-glycosidisch an Polypeptide gebunden sind und/oder
mindestens ein Glycoprotein der allgemeinen Formel (I') für einen Glycosylphosphatidylinosit(GPI)-Anker 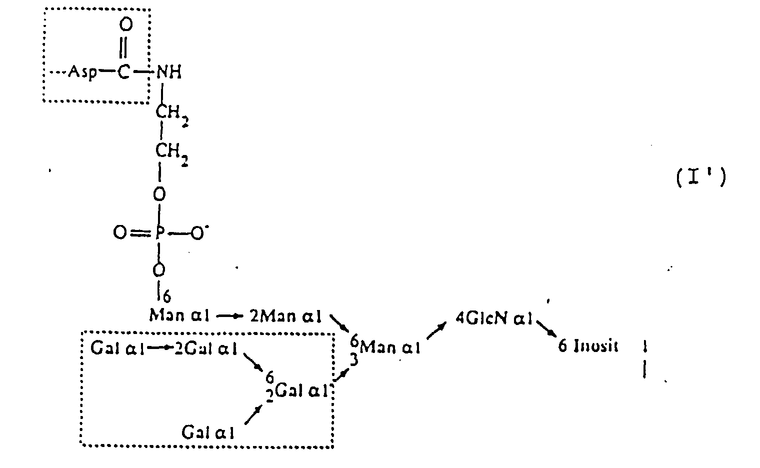 worin bedeuten:
- Gal =: Galactose,
- Man =: Mannose
- Asp =: Asparagin
- GlcN =: Glucosamin
wobei GlcN ersetzt sein kann durch ein Neuraminsäure-Vorstufen-Analogon der Formel (II) 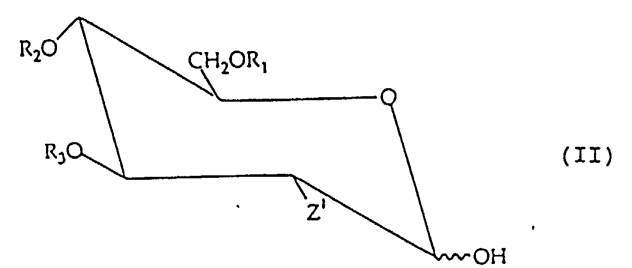 in der R₁, R₂ und R₃ die oben angegebenen Bedeutungen haben und Z' die gleichen Bedeutungen wie Z hat und neben der äquatorialen Position auch die axiale Position einnehmen kann und wobei außerdem bei äquatorialer Position von Z' der Rest -OR₂ in axialer Position stehen kann.

Die erfindungsgemäßen pharmazeutischen Mittel enthaltenden den Wirkstoff vorzugsweise in einer Menge von 0,01 bis 50 Gew.-%, insbesondere von 0,1 bis 20 Gew.-%, speziell von 2 bis 10 Gew.-%, bezogen auf das Gewicht des pharmazeutischen Mittels.

Bei den erfindungsgemäßen pharmazeutischen Mitteln handelt es sich vorzugsweise um solche
zur Stimulierung des Immunsystems, insbesondere der T-Lyphozyten, zur Infektabwehr, zur Behandlung von Immunschwächen, Tumorerkrankungen einschließlich Metastasierungsprozessen, Infektionskrankheiten (Viren, Bakterien,
Parasiten, Protozoen) und Kreislaufversagen, insbesondere Gefäßverschlüssen und Septikämien, bei Mensch und Tier;
zur Erhöhung der zytotoxischen Aktivität Natürlicher Killerzellen (NK-Zellen) zur Induktion einer Antitumor-Immunreaktion bei Mensch und Tier;
zur Steigerung der Phagozytose-Fähigkeit von Granulozyten und Monozyten zur Induktion einer Antitumor-Immunreaktion bei Mensch und Tier;
zur in vivo-Beeinflussung Neuraminsäure-abhängiger biologischer Prozesse;
zur Hemmung der Ligandenbindung an sialylierte Zelloberflächenrezeptoren (Endothelzellen, Thrombozyten, Leukozyten);
zur Hemmung der Bindung eines mikrobiellen Pathogens (Virus, Bakterium, Parasit, Protozoon) oder Toxins an die Wirtszelle über einen sialylierten Rezeptor durch in vivo-Modulation von Neuraminsäuren;
zur Stimulierung des Wachstums und der Differenzierung von menschlichen und tierischen Zellen des Immunsystems und zur Verhinderung der Adhäsion von Leukozyten, Thrombozyten und Tumorzellen an Gefäßendothelzellen;
zur biosynthetischen Herstellung von Liganden oder Rezeptoren mit modifizierter Neuraminsäure und deren Verwendung zur Kompetition physiologischer oder pathologischer Ligand-Rezeptor-Interaktionen;
zur in vitro-Beeinflussung des Infektionsverlaufs humaner Immundefizienz-Viren wie HIV-1 und HIV-2 sowie zur in vivo-Prävention der Infektion humaner Immundefizienz-Viren wie HIV-1 und HIV-2); und
zur Behandlung von parasitären Erkrankungen, insbesondere von Trypanosomiasen, Leishmaniasen, Trichomoniasis, Giardiasis, Amöbiasis, Malaria, Pneumozystose, Schistosomiasis (Bilharziose) und Echinokokkose.

Ein wichtiges Anwendungsgebiet der vorliegenden Erfindung ist die in vivo-Beeinflussung durch Neuraminsäure-Vorstufenanaloga der vorstehend angegebenen allgemeinen Formel (II) von Rezeptor-Ligand-Interaktionen, an denen Neuraminsäuren beteiligt sind.

Wie allgemein bekannt, besteht eine wichtige Eigenschaft von Kohlehydraten, die an Eiweiß gekoppelt sind, darin, daß sie dem Gesamtmolekül, d.h. dem Glycoprotein, Stabilität und Schutz vor Abbau verleihen (vgl. Ashwell und Harford (1982), Annual Review of Biochemistry 51, S. 531-554, und Tauber und Reutter (1989), Futura 2, S. 11-18 (insbesondere Schema S. 13). Es ist auch bekannt, daß Veränderungen der Oligosaccharidstruktur zu Veränderungen der biologischen Stabilität der Glycoproteine, gemessen an deren Halblebenszeit, führen. Dies gilt für die meisten untersuchen Glycoproteine.

Durch Veränderung der Oligosaccharidstruktur lassen sich daher gezielte Veränderungen der natürlich gebildeten Glycoproteine oder der gentechnologisch, biochemisch oder chemisch hergestellten Glycoproteine herbeiführen zur Bildung von Substanzen, die therapeutische Effekte ergeben. Dies ist insbesondere für die folgenden Verbindungsklassen von Bedeutung:
a) Gallensäuren
   Die erfindungsgemäßen Aminozucker (Neuraminsäure-Vorstufen-Analoga) der oben angegebenen allgemeinen Formel (II) lassen sich durch in vivo-Koppelung in Gallensäuren einführen (insbesondere in die OH-Gruppe des Stearanringes derselben) unter Bildung von Gallensäureglycosiden. Besonders vorteilhaft ist die Einführung der N-Acylhexosamine, N-Propanoyl-, N-Butanoyl-, N-Pentanoyl-, N-Hexanoyl-, N-Heptanoyl- und N-Crotonoylhexosamine, speziell ihrer -glucosamine und -galactosamine.
   Ein Großteil der Gallensäuren wird als Glycoside ausgeschieden, z.B. als N-Acetylglucosaminid. Durch Gabe von N-Acyl-D-glucosaminen wird die Bildung dieser Gallensäurenhexosaminide gesteigert. Da die Vermehrung von Gallensäuren im Blut (Bildungsort Leber) zu quälendem Juckreiz bei vielen Patienten mit Lebererkrankungen führt, wird durch die Gabe von N-Acyl-hexosaminen (wie vorstehend aufgezählt) die Gallensäuren-Eliminierung durch die Niere gesteigert und somit der Juckreiz gemindert.
b) Mucine
   Bei diesen Mucopolysacchariden, die zur Klasse der Glycosaminoglycane gehören, die eine wichtige Schutzfunktion im Bronchialsystem und im Magen-Darm-Trakt sowie im Urogenital-System ausüben, können sowohl die N-Acetyl-hexosamin-Komponenten als auch die N-Acetyl- oder N-Glycolylneuraminsäure-Komponenten teilweise oder vollständig ersetzt werden durch die erfindungsgemäßen N-Acylhexosamine bzw. deren Stoffwechselprodukte N-Acylneuraminsäuren (in vivo-Koppelung an Mucine).
   Im erstgenannten Fall lassen sich die N-Acetyl-hexosamin-Komponenten der in der Natur vorkommenden Substanzen teilweise oder vollständig ersetzen durch die erfindungsgemäßen N-Acyl-hexosamine, vorzugsweise die N-Acyl-galactosamine und -glucosamine.
   Im letztgenannten Fall können durch in vivo-Gabe der erfindungsgemäßen N-Acylhexosamine, insbesondere -mannosamine oder -glucosamine, die N-Acetyl- oder N-Glycolylneuraminsäure-Komponenten vollständig oder teilweise ersetzt werden durch die erfindungsgemäßen N-Acyl-Derivate.
   Durch Gabe der erfindungsgemäßen Aminozucker (Neuraminsäure-Vorstufen-Analoga) der oben angegebenen Formel (II) werden die physikalisch-chemischen Eigenschaften der Mucine verändert, beispielsweise bei Bronchitiden durch Verbesserung der Löslichkeit und damit Verbesserung des Schleimabhustens (Entschleimung) und im Magen-Darm-Trakt wird eine bessere (stabilere) Auskleidung mit Schleim und damit eine Verbesserung des Schutzes der Darmschleimhaut vor Schadstoffen der Nahrung und der endogen produzierten Salzsäure (Magen) erzielt.
c) Gentechnologisch oder biochemisch oder chemisch hergestellte Glycosaminoglycane wie Hyaluronsäure oder sulfatierte Glycosaminoglycane wie Heparine, Chondroitin-, Dermatan- oder Keratansulfate oder Proteoglycane wie Kollagene
   Diese Substanzen können beispielsweise eingesetzt werden zur Behandlungvon Hauterkrankungen (trockene Haut, Wunden, Neurodermitis, Ulcera). In diesen natürlichen Strukturen können die darin enthaltenen N-Acetylglucosamin- oder N-Acetyl-galactosamin-Komponenten durch die erfindungsgemäßen N-Acyl-hexosamine, insbesondere durch N-Propanoyl-, N-Butanoyl-, N-Pentanoyl-, N-Hexanoyl-, N-Heptanoyl- und N-Crotonoyl-galactosamine und -glucosamine ersetzt werden.
   Ist in diesen gentechnologisch oder biochemisch oder chemisch hergestellten Substanzen zusätzlich N-Acetyl- oder N-Glycolylneuraminsäure enthalten, so kann auch diese teilweise oder vollständig ersetzt werden auf biologischem Wege durch in vivo-Gabe der obengenannten erfindungsgemäßen N-Acyl-hexosamine, insbesondere -mannosamine und -glucosamine. Chemisch erfolgt dieser Ersatz durch Deacylierung und anschließende Reacylierung, beispielsweise mit Propionsäureanhydrid, oder biochemisch durch in vivo-Modulation: dazu wird, wie weiter oben beschrieben, z.B. der entsprechende N-Acyl-D-mannosamin-Vorläufer verabreicht (beispielsweise wird N-Propanoylmannosamin gegeben, wenn N-Propanoylneuraminsäure gebildet werden soll).
d) Gentechnologisch, biochemisch oder chemisch hergestellte Glycoproteine wie Hormone, Hyaluronsäure und ihre sulfatierten Derivate (wie oben angegeben) Hormone, wie Erythropoetin (zur Blutbildung wichtig, Mangel bei Dialyse-Patienten), Wachstumshormon (hCG = Human growth hormon), Gewebshormone, auch Mediatoren genannt, wie Interleukine (bisher sind mindestens 10 Interleukine bekannt) werden von Zellen des lymphatischen Systems und Makrophagen synthetisiert, Interleukin 6 von der Leber. Dazu gehören auch Antikörper und Interferone (α-, β-, γ-Interferon mit Untertypen) für die Behandlung von viralen Erkrankungen und Tumoren.

Auch in diesen Fällen können die darin enthaltenen N-Acetyl-glucosamin- oder N-Acetyl-galactosamin-Komponenten durch die obengenannten erfindungsgemäßen N-Acyl-hexosamine, insbesondere N-Acyl-glucosamine und N-Acyl-galactosamine, durch Verabreichung der erfindungsgemäßen Aminozucker (II) ersetzt werden.

Es können deren N-Acetyl-hexosamin-Komponenten vollständig oder teilweise ersetzt werden durch N-Acyl-hexosamine, insbesondere -glucosamine und -galactosamine. Es können aber auch die darin enthaltenen N-Acetyl-neuraminsäure-Komponenten ersetzt werden durch entsprechende N-Acylneuraminsäuren, die durch Verabreichung der erfindungsgemäßen N-Acyl-hexosamine, insbesonderemannosamine und -glucosamine, in vivo gebildet werden.

Unter den hier genannten Acylresten sind allgemein vorzugsweise Propanoyl-, Butanoyl-, Pentanoyl-, Hexanoyl-, Heptanoyl- und Crotonoylreste einschließlich der ein- oder mehrfach hydroxylierten Analoga davon zu verstehen.

Unter den hier genannten Hexosaminen sind insbesondere zu verstehen Glucosamine, Galactosamine und Mannosamine.

Viele biologische Vorgänge beruhen auf der Interaktion von Liganden (beispielsweise Hormone, Interleukine, Pharmaka, Zell-Zell-Erkennung) mit Rezeptoren auf Zelloberflächen. Auch pathogene Mikroorganismen (Viren, Bakterien, Protozoen) oder deren Toxine benutzen für ihre pathogene Wirkung häufig Zelloberflächenstrukturen als Rezeptoren für die Interaktion mit Wirtszellen. Zell-Zell-Erkennung ist wesentlich beteiligt an physiologischen und pathologischen (z.B. Autoimmunerkrankungen) Funktionen des Immunsystems, bei der Gewebedifferenzierung und bei der immunologischen Kontrolle maligner Tumorzellen.
Bei einer Vielzahl dieser Ligand-Rezeptor-Interaktionen sind Kohlenhydrat-Strukturen wesentlich beteiligt. Eine besondere Rolle spielen dabei terminale Neuraminsäuren auf diesen Kohlenhydrat-Strukturen: Beispielsweise benutzt das Influenzavirus einen sialylierten Zelloberflächen-Rezeptor. Zur Aktivierung von T-Lymphozyten sind sialylierte Differenzierungsantigene notwendig. Des Weiteren ist unterschiedliche Zelloberflächen-Sialylierung als Determinante für Progession und Metastasierung maligner Tumoren beschrieben worden.

Eine Möglichkeit, Neuraminsäure-abhängige biologische Prozesse zu beeinflußen, besteht in der Verwendung nicht-toxischer Neuraminsäure-Vorstufen-Analoga, die von Zellen aufgenommen werden und nach Metabolisierung anstelle von physiologischer Neuraminsäure in Kohlenhydrat-Strukturen eingebaut werden. Die eingeführte Modifikation der Neuraminsäure kann Ligand-Rezeptor-lnteraktion abschwächen, verstärken oder unbeeinflußt lassen.
Je ein Beispiel für Abschwächung bzw. Verstärkung einer Rezeptor-Ligand-Interaktion wird im Folgenden beschrieben:
A. Reduktion der Infizierbarkeit von humanen B-Lymphomzellen (BJA-B) für das lymphotrope Papovavirus (LPV) durch Vorbehandlung mit N-Propanoyl-D-Mannosamin oder N-Butanoyl-D-Mannosamin.
   Der von LPV auf BJA-B-Zellen benutzte Rezeptor ist Neuraminsäure-abhängig, da Behandlung der Zellen mit *Vibrio cholerae* Neuraminidase die Virusbindung und die Infizierbarkeit um mehr als das Fünffache reduziert (eigene unveröflentlichte Ergebnisse).
B. Verstärkung der Infizierbarkeit von Affen-Nieren-Epithelzellen (Vero) für das humane Poylomavirus BK (BKV) durch Vorbehandlung mit N-Propanoyl-D-Mannosamin. Für die Infektion von Vero-Zellen durch BKV ist eine Zelloberflächen-Neuraminsäure wesentlich, da Präinkubation von Vero-Zellen mit Neuraminidase, die Infizierbarkeit reduziert (Sinibaldi et al., Arch. Virol. 113 (1990), 291-296).

### MATERIAL UND METHODEN

### 1. Verwendete Abkürzungen

- A: Absorption
- BKV: BK Virus
- DAPI: 4',6-Diamidino-2-phylindol
- DMEM: Dulbecco's modifiziertes Eagle Medium
- DTT: 1,4-Dithiothreitol
- EBV: Epstein-Barr Virus
- ELISA: Enzymgekoppelter Immunosorbent Test
- FITC: Fluorescinisothiocyanat
- FKS: Fötales Kälberserum
- LPV: Lymphotropes Papovavirus
- LRSC: Lisamin-Rhodamin-lsothiocyanat
- mAk: Monoklonaler Antikörper
- OD: Optische Dichte
- PBS: Phosphat-gepufferte Salzlösung
- rpm: Umdrehungen pro Minute
- RT: Raumtemperatur
- SD: Standardabweichung
- SV40: Simian Virus 40
- TMB: Tetramethylbenzidin
- TRITC: Tetra-Rhodamin-lsothiocyanat
- VP: Virales Strukturprotein
- FACS: Fluoreszenz-aktivierter Cellsorter
- Scan: Scanner

### 2. Zellkulturmedien

Als Zellkulturmedien wurden RPMI 1640 und DMEM Medium verwendet, die von Biochrom, Berlin.als Trockensubstanz bezogen wurden. Für den Gebrauch wurden sie nach Herstellerangaben in bidestilliertem, pyrogenireiem H₂O aufgenommen und sterilfiltriert Zugesetzt werden 10% FKS [30 min bei 56°C inkubiert zur Inaktivierung des Komplementsystems], 100 U/ml Pennicillin, 100 µg/ml Streptomycin und 2 mM L-Glutamin.

### 3. Puffer

- *PBS*:
   125 mM NaCl (7,2 g); 18,4 mM Na₂HPO₄ (3,14 g); 10,9 mM KH₂PO₄ (1,42 g); H₂O ad 1 l (pH 7,2)
- "*Extraktionspuffer*" zur Herstellung von LPV- und BKV-lmpfvirus aus infizierten Kulturen:
   50 mM HEPES pH 7,4; 1 mM MgCl₂; 0,5 mM CaCl₂; 1 mM DTT; 1mM PMSF; 2,5 µg/ml Amphotericin B; 200 µg/ml Gentamycin; 200 U/ml Penicillin; 200 µg/ml Streptomycin.
   DTT, PMSF, Amphotericin B, Gentamycin, Penicillin und Streptomycin werden dem autoklavierten Puffer vor Gebrauch frisch zugesetzt.

### 4. Zellinien

Die humane B-Lymphom-Zellinie BJA-B (Menezes et al., Biomedicine 22 (1975), 276-284) wurde als LPV-suszeptible Zellinie benutzt. Diese Zellinie ist erhältlich bei der European Collection of Animal Cultures (ECACC), Porton Down, Salisbury (GB), hinterlegt gemäß den Bedingungen des Budapester Vertrags unter der Nummer 86081105. Als BKV-suszeptible Zellinie wurde die Affen-Nieren-Epithelzellinie Vero benutzt. Diese Zellinie ist erhältlich bei der American Type Culture Collection (ATCC), Rockville, Maryland (USA) unter der Nummer ATCC CRL 1587.

### 5. Zellkultur

Alle Zellkulturarbeiten wurden unter einer Sicherheitswerkbank (L II) durchgeführt. Die Zellen wurden in Brutschränken bei 37°C mit 5% CO₂ und 95% Luftfeuchtigkeit gehalten. BJA-B Zellen wurden in Glas-Erlenmeyerkolben mit Aluminiumfoliendeckel kultiviert. Bei einer Dichte von 2x10⁵ bis 1x10⁶ Zellen pro ml befanden sich die Zellen in der logarithmischen Wachstumsphase. Hatten sie eine stationäre Wachstumsphase erreicht, wurden sie mit frischem Medium gefüttert und dabei 1:3-1:10 vedünnt (Forbes et al., Mol. Cell. Probes 2 (1988), 245-253). Die adhärent wachsenden Monolayer-Zellinie Vero wurde in Zellkultur-Plastikflaschen gehalten. Bei Erreichen der Konfluenz wurde das Medium abgenommen und der Zellrasen mit wenigen ml Trypsin-Lösung [0,1% Trypsin; 0,1% EDTA in H₂O] gespült. Nach kurzem Einwirken von Trypsin bei 37°C lösten sich die Vero Zellen vom Untergrund ab. Dann wurden sie 1:3-1:20 mit frischem Medium verdünnt (Mühlbach et al., Virology 186 (1992), 65-73).

Zur Zellkonzentrationsbestimmung wurden 100 µl suspendierter Zellen mit 100 µl Trypanblau-Lösung [0,25% Trypanblau, gelöst in PBS] gemischt und einige Tropfen zwischen Deckglas und Kammer einer Neubauer-Zählkammer pipettiert. Tote Zellen färbten sich blau an und konnten von hell-leuchtenden lebenden Zellen im Lichtmikroskop unterschieden werden. Die Summe der Zellzahlen von zwei der vier Quadranten multipliziert mit 10⁴ ergab die Zellzahl pro ml der Ausgangskultur.

### 6. Antiseren und monoklonale Antikörper

Benutzt wurden ein polyklonales Hamster-α-LPV-T-Antigen Serum, ein polyklonales Kaninchen-α-LPV-VP-Serum und monoklonaler Antikörper (mAk) 456-1 aus Maus-Aszites gegen LPV-VP1 von M. Pawlita, Heidelberg; außerdem wurden ein Maus-mAk gegen SV40-T-Antigen (mAk SV1-3H9) von F. Mehnert, Bochum, sowie ein Kaninchen-Antiserum gegen BKV Partikel von G. Noss, Rehlingen-Siersburg, verwendet.

### 7. Herstellung von Impfvirus

### 7.1. Herstellung von LPV-lmpfvirus

Das lymphotrope Papovavirus (LPV) ist bei der American Type Culture Collection unter der Nummer ATCC VR-961 erhältlich. Zu 2x10⁷ BJAB Zellen in Kultur werden 200-300 µl einer LPV-lmpfvirus Lösung gegeben. Die Kultur wird alle drei Tage 1:5 mit frischem Medium expandiert und ab dem 6. Tag wird über indirekte Immunfluoreszenz der Prozentsatz Virus-infizierter Zellen bestimmt; liegt dieser zwischen 30% und 60% so wurde die Kultur geerntet. Dazu wurden die Zellen für 15 min bei 600 g abzentrifugiert, in PBS gewaschen und das Zellsediment zur Zellyse bei -20°C eingefroren. Zur Virusextraktion wird das Zellsediment in kaltem "Extraktionspuffer" (1/20 des Zellkulturvolumens) resuspendiert. Die Suspension wird unter häufigem Aufwirbeln (Vortexen) für 60 min auf Eis gehalten bevor die Zelltrümmer mit 2500 g für 10 min bei 4°C abzentrifugiert werden. Das verbleibende Sediment wird noch dreimal mit 1/60 des ursprünglichen Zellkulturvolumens jeweils für 5-10 min nachextrahiert. Nachfolgend werden die vier Überstände vereinigt. Durch Titration auf BJA-B Zellen wurde die Infektiosität der LPV-Suspension bestimmt und das Impfvirus bis zur Verwendungbei -20°C eingefroren.

### 7.2. Herstellung von BKV-lmpfvirus

Das humane Polyomavirus BK (BKV) ist bei der American Type Culture Collection unter der Nummer ATCC VR-837 erhältlich. BKV-lmpfvirus wurde aus infizierten und für ca. 3 Wochen kultivierten Vero Zellen gewonnen. Die Zellen wurden mit Trypsin abgelöst und 15 min bei 600 g abzentrifugiert in PBS gewaschen und das Zellsediment zur Zellyse bei -20°C eingefroren. Das Zellsediment wurde zweimal einem Frieren-Tauen Vorgang unterzogen und zur Virusextraktion in kaltem "Extraktionspuffer" (1/20 des Zellkulturvolumens) resuspendiert. Die Suspension wurde unter häufigem Aufwirbeln (Vortexen) für 60 min auf Eis gehalten, bevor die Zelltrümmer mit 2500 g rpm für 10 min bei 4°C abzentrifugiert wurden. Nachfolgend wurde die Infektiosität des Überstands auf Vero Zellen austitriert das Impfvirus bis zum Gebrauch bei -20°C eingeforen.

### 8. Behandlung von Zellen mit Neuraminidasen und nachfolgende Virus-Infektion

1,5x10⁶ BJA-B Zellen wurden zweimal in PBS gewaschen, 1 h in PBS bei 37°C gehalten und dann mit 20 mU Neuraminidase ( von *vibrio cholerae)* in 100 µl PBS (0,2 U/ml) auf einem Schüttler für 60 min bei 37°C inkubiert. Nach einmaligem Waschen (4°C, 400 g, 10 min) in PBS wurden die Zellen erneut mit Neuraminidase (0,2 U/ml) unter den gleichen Bedingungen inkubiert. Nach weiteren 60 min werden die Zellen in kaltem Medium aufgenommen und 10 min bei 4°C und 2600 rpm abzentrifugiert. Das Zellsediment wurde in 400 µl einer LPV-lmpfvirus Suspension aufgenommen und bei 4°C für 3 h unter mehrmaligem Aufwirbeln inkubiert. Das nicht-zellgebundene Virus wurde durch einmaliges Waschen mit Medium entfernt und die Zellen nachfolgend in 2 ml frisches Medium aufgenommen und für 48 h kultiviert.
Zu ca. 50% konfluent gewachsene Vero Zellen auf Deckgläsern wurden mit PBS gewaschen und wie oben beschrieben mit Neuraminidase (von *vibrio cholerae)* für ingesamt 2 h inkubiert. Die Enzymlösung wurde mit Medium abgespült und die Zellen nachfolgend mit BKV-lmpfvirus für 3 h bei 4°C infiziert. Nicht zellgebundenes BKV wurde von Vero Zellen durch dreimaliges Spülen mit Medium abgewaschen. Nach 48 h Kultur bei 37°C wurde der Prozentsatz Virus-infizierter Zellen mittels indirekten Immunfluorezenz bestimmt werden.

### 9. Behandlung von Zellen mit N-Acyl-D-Mannosaminen und nachfolgende LPV- oder BKV-Infektion

BJA-B Zellen wurden in frischem Medium aufgenommen zu Zellkonzentrationen von 1x10⁶/ml für Behandlungsdauern von 3 h und 2x10⁵/ml für 48 h Inkubation. Die Konzentration der in PBS gelösten Neuraminsäure-Vorstufenanaloga wurde zwischen 0,01 und 10 mM in Kultur variiert. Monolayerzellen wurden dünn auf Deckgläschen ausgesät, so daß sich ein konfluenter Zellrasen erst nach etwa vier Tagen einstellte.
Nach Behandlung mit den Neuraminsäure-Vorstufenanaloga wurden die Zellen in PBS gewaschen und für 3 h bei 4°C mit Impfvirus infiziert. Mit LPV-infizierte BJA-B Zellen wurden dann einmal mit Medium gewaschen und abzentrifugiert, mit BKV-infizierte Vero Zellen wurden dreimal mit Medium gespült. Nach weiteren 48 h in Kultur konnte der Prozentsatz Virus-infizierter Zellen in der indirekten Immunfluorezenz bzw. die LPV-Permissivität bestimmt werden.

### 10. Messung von Virus-Bindung und -Infektion

### (i). Bestimmung des Grades der Virusinfektion

Der Grad der LPV-Infektion in Kultur wurde sowohl über eine Bestimmung des Prozentsatzes Virus-Antigen-produzierender Zellen mittels indirekter Immunfluoreszenz (Forbes et al, a.aO.) als auch über eine Quantifizierung des viralen Hauptstrukturproteins LPV-VP1 mittels ELISA (10.2.3.) relativ zur Gesamtproteinmenge eines Extraktes infizierter Zellen ermittelt. Dieser Quotient (ausgedrückt als ng LPV-VP1 pro mg Gesamtprotein) wird nachfolgend als LPV-Permissivität bezeichnet.
Der Grad der BKV-Infektion wurde ebenfalls über indirekte Immunfluoreszenz unter Verwendung eines Kaninchen-anti-BKV-Partikel-Serums bestimmt.

### (ii). LPV-Bindungstest

Die Bindung von LPV-Partikeln an BJA-B-Zellen wurde wie unter 10.3. beschrieben gemessen.

### 10.1. Indirekte Immunfluoreszenz

Zur Bestimmung des Prozentsatzes Virus-infizierter Zellen wurden 5x10⁵ BJA-B Zelllen 48 h nach Infektion abzentrifugiert und in 50 µl PBS resuspendiert (1x10⁴ Zellen/µl). 10 µl wurden auf ein Feld eines poly-L-Lysin-beschichteten Adhäsions-Objektträgers ausgebracht (BioRad adhesion slides, BioRad, München). In einer feuchten Kammer konnten sich die Zellen in 30 min auf den poly-L-Lysin-beschichteten Untergrund absetzen. Nicht angeheftete Zellen wurden mit PBS abgespült und die Objektträger anschließend in einem "kaltem" Aceton/Methanol Gemisch [Im Verhältnis 1:1, bei -20°C aufbewahrt] für 5 min fixiert.
Vero Zellen wurden auf Deckgläschen (Durchmesser 10 mm) ausgesät werden, wuchsen dort an und wurden nach einmaligem PBS-Waschen, wie oben beschrieben, fixiert werden.
Die fixierten Zellpräparate wurden mit je 10-30 µl/Feld monoklonalen oder polyklonalen Erstantikörpern bei 37°C in einer feuchten Kammer für 45-60 min inkubiert. Die Objektträger wurden anschließend in einer mit PBS gefüllten Schale mit Hilfe eines Magnetrührers vorsichtig für 5 min gewaschen. Als Zweitreagentien dienten komerziell erhältliche Fluorochrom-gekoppelte (FITC, TRITC, LRSC) Antiimmunoglobuline (1:50-1:100 verdünnt in PBS). Den Zweitreagentien wurde DAPI zur Kernfärbung zugesetzt. Nach einer weiteren 45 bis 60-minütigen Inkubation unter Lichtausschluß und nachfolgendem Waschen wurden die Präparate mit einer Eivanol-Lösung [20 g Eivanol (Polyvinylalkohol), 160 ml PBS, 80 ml Glycerin. Die Elvanol-Lösung wurde bei 80°C gehalten bis sich das Elvanol vollständig gelöst hat, dann portionsweise abgefüllt und autoklaviert] und Deckgläsern (24x60 mm) luftblasenfrei eingebettet und bei 4°C lichtgeschützt gelagert.
Die Fluoreszenzmikroskopie wurde an einem Vanox-T Mikroskop von Olvmpus, Tokio, Japan durchgeführt

### 10.2.1. Extraktion infizierter Zellen zur Bestimmung der LPV-Permissivität

Die Zellen (ca. 3-4 x 10⁶) wurden 48 h nach Infektion geerntet, in PBS gewaschen und das Zellsediment zur Zellyse für mindestens einen Tag bei -20°C eingefroren. Die Virusextraktion wurde, wie unter 7.1. beschrieben, durchgeführt: Das Zellsediment wurde in 400 µl kaltem "Extraktionspuffer" resuspendiert und unter häufigem Aufwirbeln für 60-90 min auf Eis gehalten, bevor die Zelltrümmer mit 4500 g für 10 min bei 4°C abzentrifugiert wurden. Der Virus-haltige Überstand wurde auf seine Protein-(10.2.2.) und LPV-VP1-Konzentration (10.2.3.) analysiert und daraus die LPV-Permissivität der infizierten Zellen errechnet.

### 10.2.2. Protein-Konzentrationsbestimmung des Zellextrakts

Die Proteinkonzentration des Extrakts infizierter Zellen wurde mit dem "BioRad-Micro-Assay" entsprechend den Herstellerangaben bestimmt. Es wurde ein solches Volumen der Proteinlösungen eingesetzt, daß die Blaufärbung derjenigen von 10 oder 20 µg des vom Hersteller mitgelieferten Proteinstandards entsprach. Die genaue Proteinkonzentration der Proben wurde aus der Standardkurve intrapoliert.

### 10.2.3. LPV-VP1-ELISA

Der affinitätsgereinigte mAk 456-1 gegen LPV-VP1 wurde 1:10⁴ in Kopplungspuffer [0,05 M Na₂CO₃ (pH 9,6)] verdünnt und jeweils 100 µl pro Napf einer ELISA-Platte (96-Napf Platte, NUNC, Wiesbaden) zugegeben. Nach Lagerung bei 4°C über Nacht wurden die Platten mit Waschpuffer (PBS mit 0,05% Tween 20) in einem Ultrawash II Gerät (Dynatech, Denkendorf) durchgespült (5x300 µl pro Napf), um nicht-gebundene Antikörper zu entfernen. Das Blocken noch freier Bindungsstellen wurde mit 200 µl pro Napf einer 0,2% Gelatine Lösung in PBS mit 0,1% Natriumazid bei RT für 2 h durchgeführt. Die Platten konnten dann bis zur Benutzung für mehrere Wochen bei 4°C aufbewahrt werden.
(1) Die zu vermessenden LPV-Suspensionen aus extrahierten Zellen wurden in PBS 1:2 bis 1:2000 verdünnt und 100 µl pro Napf eingesetzt. Aus einer gereinigten LPV-Partikel Präparation (5 µg LPV-VP1/µl) wurde eine Verdünnungsreihe von 12,5 pg bis 1,6 ng LPV-VP1 pro 100 µl pro Napf angesetzt, die Standardkurve diente. Für jede Probe wurde eine Zweifachbestimmung durchgeführt Die Inkubation wurde bei 37°C für 60 min durchgeführt.
(2) Die Platte wurde mit Waschpuffer gründlich gespült (9x300 µl pro Napf).
(3) Das polyklonale Kaninchen Serum gegen LPV-VP wurde 1 : 2500 in Waschpuffer verdünnt und jeweils 100 µl pro Napf als Zweitantikörper zugegeben. Die Inkubation fand wie oben beschrieben bei 37°C für 60 min statt.
(4) Die Platte wurde erneut mit Waschpuffer gespült (9x300 µl pro Napf).
(5) Peroxidase-gekoppeltes Ziege anti-Kanninchen IgG wurde in Waschpuffer verdünnt (1 : 5000) und 100 µl pro Napf der ELISA-Platte zugegeben. Die Inkubation fand bei 37°C für 60 min statt.
(6) Nach erneutem Waschen (wie (2) und (4)) wurden pro Napf 100 µl Substratlösung [9,9 ml 0,1 M Natriumacetatpuffer (pH 6,0), 100 µl Tetramethylbenzidin (TMB)-Substrat, 2 µl 37% H₂O₂] zupipettiert. Die im Napf befindliche Peroxidase setzte diese Substrat um, es entstand eine bläuliche Färbung. Nach 10-20 min wurde die Reaktion durch Zugabe von 1 M H₂SO₄ (50 µl pro Napf) gestoppt und bei einer Wellenlänge von 450 nm spektrophotometrisch vermessen. Die Berechnung der LPV-VP1 Standardkurve und Umrechnung der OD-Werte der Proben in ng LPV-VP1 wurde mit einem Programm des Photometer Titertek Multiskan Plus MKII von Flow Laboratories, Meckenheim durchgeführt.

### 10.3. LPV-Bindungstest an BJA-B Zellen

Gewaschene Zellen (1x10⁶) wurden mit gereinigten LPV-Partikeln (10 ng LPV-VP1) für 30 min bei 37°C in 500 µl PBS (2 x 10⁶ Zellen/ml) inkubiert. Nach Zentrifugation der Zellen bei 12500 rpm für 2 min wurde das ungebundene, im Überstand befindliche Virus im LPV-VP1-ELISA quantifiziert. LPV-Bindung wurde als Prozentsatz des zellassoziierten Virus relativ zur eingesetzten Gesamtvirusmenge (= 100%) bestimmt.

### ERGEBNISSE

### A. Hemmung der LPV-lnfizierbarkeit durch Vorbehandlung von BJA-B-Zellen mit N-Propanoyl- oder N-Butanoyl-D-Mannosamin.

Bei einer Endkonzentration der Analoga von 10 mM und 48 h Vorbehandlungszeit von BJA-B-Zellen war sowohl der Prozentsatz LPV-infizierter Zellen in der indirekten Immunfluoreszenz als auch die LPV-Permissivität zwischen 89 und 95% reduziert. Bei gleicher Behandlungsdauer wurde mit 0.4 mM N-Propanoyl-D-Mannosamin eine 50% ige Reduktion der LPV-Permissivität erreicht.
In Zellen, die für 48 h mit 5 mM N-Butanoyl-D-Mannosamin vorbehandelt waren, wurde eine 80 %ige Reduktion der LPV-Bindung beobachtet.

### B. Verstärkung der BKV-Infizierbarkeit von Vero-Zellen durch Vorbehandlung mit N-Propanoyl-D-Mannosamin.

Bei einer Endkonzentration von 10 mM und 48 h Vorbehandlung von Vero-Zellen wurde ein Anstieg der Zahl BKV-infizierter Zellen um das Siebenfache erreicht. Behandlung der mit Analogon vorinkubierten Zellen mit *Vibrio cholerae* Neuraminidase (200 mU/ml, 2 h, 37°C) führte zu einer über 80 %igen Reduktion dieser Infizierbarkeit.

Ein weiteres wichtiges Anwendungsgebiet der Erfindung ist die Beeinflussung der Replikation des humanen Immundefizienz-Virus Typ 1 (HIV-1) in MT-4 Zellen durch Neuraminsäure-Vorstufen-Analoga (Aminozucker) der vorstehend angegebenen allgemeinen Formel (II).

### 1. Material und Methoden

Die humane T-Zellinie MT-4, die für HIV-1 hoch-suszeptibel ist (Harada et al., Science 229 (1985), 563-566), wurde in RPMI 1640 unter Zusatz von 10% Hitze-inaktiviertem FKS, 100 U/ml Pennicillin, 100 µg/ml Streptomycin und 2 mM L-Glutamin, wie oben beschrieben, kultiviert unter Labor-Sicherheitsbedingungen der Stufe L3.
HIV-1 (Stamm HTLV-III_{B})-Impfvirus (Infektiosität: 1x10⁶ IU/ml) wurde aus dem Überstand infizierter MT-4 Zellen gewonnen wie beschrieben in Popovic et al., 1984 (Science 224, 497-501). Das HIV-Impfvirus wurde aliquotiert und bei -70°C gehalten. HIV-Stamm HTLV-III_{B} ist bei der Division of AIDS, National Institute of Allergy and Infectious Diseases, 6003 Executive Boulevard, Bethesda, MD 20892 (catalog number 398) erhältlich.
5x10⁵ MT-4 Zellen wurden in 2 ml Medium in 12-Napf Platten für eine Behandlungsdauer von 40 h mit N-Acyl-D-Mannosaminen kultiviert. Als Konzentration der in PBS gelösten Neuraminsäure-Vorstufen-Analoga wurden 2.5 und 5 mM in Kultur benutzt. Nachfolgend wurden die Zellen in PBS gewaschen, gezählt und 1x10⁶ lebende Zellen in 2 ml Medium aufgenommen und mit 100 µl HTLV-III_{B}-Impfvirus für 3 h bei 37°C infiziert. Nach einmaligem Waschen in 2 ml PBS wurden die Zellen erneut in Medium aufgenommen und für weitere 24 h kultiviert. In Überständen dieser Kulturen wurden mit Hilfe eines ELISA (Vironostika HIV-Antigen ELISA Mikrosystems, Organon, Eppelheim) nach Herstelllerangaben die HIV-1-Antigen Konzentration bestimmt.

### 2. Ergebnisse

Die Behandlung von MT-4 Zellen mit N-Butanoyl-D-Mannosamin (40 h) führte bei einer Konzentration des Neuraminsäure-Vorstufen-Analogons von 5 mM zu einer Reduktion von HIV-Antigen im Überstand von 97%, bei einer Konzentration des Neuraminsäure-Vorstufen-Analogons von 2,5 mM zu einer Reduktion von HIV-Antigen im Überstand von 85% gegenüber unbehandelten Zellen.
Als Kontrolle diente die Behandlung von MT-4 Zellen mit N-Acetyl-D-Mannosamin (40 h, 5 mM). Hier war die HIV-Antigen Konzentration im Überstand um 21% gesteigert gegenüber unbehandelten Zellen.

### Angewendete Methoden

### In vitro-Stimulation von Lymphozyten

Aus heparinisiertem Blut von Tumorpatienten bzw. "buffy coat" von gesunden Probanden wurden periphere Blutlymphocyten (PBL) durch eine Dichtegradientenzentrifugation nach Böyum gewonnen.
Dazu wurden 15ml Ficoll-Paque (Dichte: 1,077 g/ml) im Zentrifugen-Röhrchen (50 ml) mit 30 ml Blut, das vorher 1:2 bis 1:3 mit physiologischer Kochsalzlösung verdünnt wurde, vorsichtig überschichtet.. Es wurde bei 600g 30min zentrifugiert. Bei der Zentrifugation wandern die Erythrocyten und Granulocyten durch die Ficoll-Schicht, während sich die mononukleären Blutleukocyten unmittelbar über dem Trennmedium anreichern. Diese Zellschicht wurde entnommen, in Kochsalzlösung aufgenommen und fünfmal gewaschen (200g, 5min). Anschließend wurden die Zellen in RPMI-1640-Medium mit 10% FKS, 10⁻⁵mol/l 2-Mercaptoethanol und 2 mmol/l L-Glutamin aufgenommen und in Plastik-Kulturschalen im Brutschrank 16h inkubiert, um adhärente Monocyten abzutrennen. Die Suspension der nicht-adhärenten, peripheren Blutlymphocyten wurde auf eine Zelldichte von 5 x 10⁶/ml verdünnt.
Zur Überprüfung der Vitalität der Zellen wurde die Trypanblaufärbung angewendet. Zu einer Zellsuspension (20µl)wurden 80µl einer 0,1 %igen Trypanblaulösung gegeben. Nach Mischung des Testansatzes erfolgte die Auszählung vitaler Zellen in der Neubauer-Zählkammer.
Zur Stimulierung von Lymphocyten wurde Concanavalin A (4µg/ml) zur Zellsuspension gegeben, eine maximale Wirkung trat nach 72h ein. Für eine Kurzzeitaktivierug wurde Con A (4 µg/ml) 1h zur Zellsuspension gegeben. Danach wurden die Zellen zweimal gewaschen (200g, 5min).

### Verwendete Zellinien:

ATCC CCL86 (Raji)
ATCC CCL 213 (Daudi)
ATCC CCL 229 (LoVo)
ATCC CCL 240 (HL-60)
ATCC CCL 243 (K-562)
ATCC CRL 1582 (Molt-4)
ATCC CRL 1593 (U-937)
ATCC HTB 22 (MCF-7)
ATCC HTB 38 (HT-29)

### Aufnahme und Einbau der Wirkstoffe

Die verschiedenen Zuckeranaloga wurden der Zellsuspension in einer Konzentration zwischen 0,05 mmol/l und 50mmol/l in Mikrotiterplatten zugesetzt. Die Inkubationszeit betrug 1 h bis 72h.

### Bestimmung der Zellproliferation

Zur Bestimmung der Zellproliferation wurde der ³H-Thymidin-Test verwendet. Dieser Test basiert auf der Bestimmung der ³H-Thymidin-Einbaurate in die DNS, die sich proportional zur Zellzahl verhält.
Dazu erfolgte die Kultivierung der Zellen in Mikrotestplatten. In jede Vertiefung wurden 200µl der entsprechenden Zellsuspension gegeben. Jeweils 16h vor Beendigung der Inkubation wurden zu jedem Testansatz 0,037 MBq ³H-Thymidin pro Vertiefung zugesetzt. Anschließend wurden jeweils 100 µl Zellsuspension auf Filterplättchen gegeben. Die luftgetrockneten Plättchen wurden je zweimal (30min) mit 10 %iger bzw. 5 %iger Trichloressigsäure sowie 50 %igem Ethanol gewaschen und luftgetrocknet. Die Messung der ³H-Thymidinaktivität erfolgte im Flüssigkeitsszintillationszähler.

### Adhäsionsassay an extrazellulärer Matrix

Der Adhäsionsassay wird in 96-well-Mikrotiterplatten durchgeführt, die mit verschiedenen Bestandteilen der extrazellularen Matrix (Fibronectin, Vitronectin, Collagen, Laminin) beschichtet sind. In Abhänigkeit der einzusetzenden Zuckeranaloga soll die Adhäsionsfähigkeit von Zellen untersucht werden. Zur Bestimmung der relativen Anzahl gebundener Zellen werden der MTT-Test, der MUH-Test und eine Kristallviolettfärbemethode herangezogen.
Der MUH-Test beruht auf der Hydrolyse des fluorogenen Substrats MUH (4-Methylumbelliferylheptanoat) durch Esterasen (Dotsika et al., 1987).
Der MTT-Test basiert auf der Umwandlung von Tetrazoliumsalz in farbiges Formazan durch Dehydrogenasen aktiver Mitochondrien (Alley et al., 1988).

### Adhäsionsassays an Endothelzellen

Endothelzellbeschichtete Mikrotiterplatten werden mit ⁵¹Cr-markierten Tumorzellen inkubiert. Zur Bestimmung der relativen Anzahl gebundener Tumorzellen wird die Aktivität des Lysates gemessen.

### Untersuchung der Modullerbarkeit von Adhäsionsmolekülen

Der Nachweis der Adhäsionsrezeptoren bzw. deren Modulierbarkeit durch Zuckeranaloga erfolgte durchflußcytometrisch am FACScan mit monoclonalen Antikörpern.

### Invasionsassay

Zur Bestimmung des invasiven Potentials von Tumorzellen wurden Boyden-Kammern verwendet, deren obere Kammer mit Bestandteilen der extrazellulären Matrix beschichtet sind (Repesch, 1989). Der Einfluß synthetischer Zuckeranaloga auf das Migrationsverhalten wurde in dieser Anordnung untersucht.

### Bestimmung der NK-Zellaktivität

Die Bestimmung der NK-Zellaktivität peripherer mononukleärer Blutleukocyten erfolgte in einem Cytotoxicitätstest mit ⁵¹Cr-markierten Targetzellen (K562-Zellen). Der Versuch wurde in Mikrotestplatten durchgeführt. Die ⁵¹Cr-Freisetzung wurde bei verschiedenen Effektor-Targetzell-Verhältnissen (50:1; 25:1; 12.5:1; 6,25:1) bestimmt.
Zur Bestimmung der "maximalen Freisetzung" von ⁵¹Cr wurde den Targetzellen anstelle der Effektorzellen Medium mit einem Gehalt von 2% Triton X-100 zugesetzt. Als "spontane Freisetzung" bezeichnet man diejenige Menge an ⁵¹Cr, die die Targetzellen abgeben, wenn anstelle der Effektorzellen lediglich Kulturmedium zugesetzt wird.
Mit Hilfe eines Rechnerprogramms (Pross et al., 1984) für nichtlineare Regressionsanalysen wurden die lytischen Einheiten berechnet. Die Regressionskurve beschreibt die Abhänigkeit der spezifischen ⁵¹Cr-Freisetzung vom Effektor-Targetzell-Verhältnis und somit von der Menge der Effektorzellen. Mit Hilfe dieser Regressionskurve wurden die lytischen Einheiten bestimmt. Eine lytische Einheit (LU) entspricht derjenigen Effektorzahl, die notwendig wäre, 30% spezifische ⁵¹Cr-Freisetzung zu bewirken.

### Phagocytosetest

Monocyten wurden mit Zuckeranaloga behandelt. Als Phagocytosepartikel dienten neben Anti-D-sensibilisierten Erythrocyten Neuraminidase-behandelte Schaferythrocyten. Ein zweiter Teil bewertete die Fähigkeit von Granulocyten, innerhalb von einer Stunde Latexpartikel aufzunehmen.

### In vivo-Versuche

Am Beispiel experimenteller Hepatome, insbesondere am Morris-Hepatom 7777, wurde in vivo geprüft, ob es möglich ist, durch Behandlung von Ratten mit Zuckeranaloga und anschließender Injektion von Hepatomzellen deren Wachstum zu hemmen bzw. zu verhindern. Andererseits wurden Hepatomzellen mit Zuckeranaloga behandelt werden und anschließend Ratten appliziert, um deren Tumorwachstum zu testen.

### LITERATUR:

Alley, M. C., Scudiero, D. A., Monks. A., Hursey, M. L., Czerwinski. M. J.. Abbott. B. J., Mayo, J. G., Shoemaker, R. M., Boyd, M. R.
   Feasibility of drug screening with panels of human tumor cell lines using a microculture tetrazolium assay.
   Cancer Res., 48, 589-601 (1988)
Böyum. A.
   Separation of Blood Leukocytes, Granulocytes and Lymphocytes.
   Tissue Antigens, 4, 269-274 (1974)
Dotsika, E. N.,Sanderson, C. J.
   A fluorometric assay for determining cell growth in lymphocyte proliferation and lymphokine assays.
   J. Immunol. Meth., 105, 55-62 (1987)
Pross, H. F., Marown, J. A.
   The standardisation of NK cell assays for use in studies of biological response modifiers.
   J. Immunol. Meth., 68, 235-249 (1984)
Repesch, L. A.
   A new in vitro assay for quantitating tumor cell invasion.
   Invasion Metastasis, 9, 192-208 (1989)

## Patentansprüche

1. Glycoproteine der allgemeinen Formel (I) 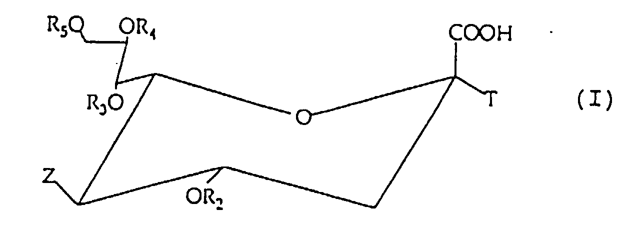 worin bedeuten:
Z -NHR, worin R den Pentanoyl-, Hexanoyl-, Heptanoyl- oder Crotonoylrest einschließlich seiner ein- oder mehrfach hydroxylierten Analoga darstellt
R₁, R_{1'} , R_{1"} , R₂, R₃, R₄ und R₅, die gleich oder verschieden sein können, jeweils Wasserstoff, einen linearen oder verzweigten Alkylrest mit 1 bis 20 Kohlenstoffatomen (CₙH₂ₙ₊₂, n = 1 bis 20), vorzugsweise 1 bis 7 Kohlenstoffatomen; einen linearen oder verzweigten Alkenylrest mit 3 bis 20 Kohlenstoffatomen (CₙH₂ₙ, n = 3 bis 20, Position der Doppelbindung an Cₙ n = 2 bis 19), vorzugsweise 3 bis 10 Kohlenstoffatomen; einen linearen oder verzweigten Alkinylrest mit 3 bis 20 Kohlenstoffatomen (CₙH₂ₙ₋₂, n = 3 bis 20, Position der Dreifachbindung an Cₙ n = 2 bis 19), vorzugsweise 3 bis 10 Kohlenstoffatomen; einen Alkenyl- bzw. Alkinylrest mit 2 oder mehr Doppelbindungen bzw. Dreifachbindungen mit 4 bis 20, vorzugsweise 7 bis 12 Kohlenstoffatomen; einen Arylrest mit 6 bis 20 Kohlenstoffatomen, vorzugsweise einen Phenylrest; einen linearen oder verzweigten, gesättigten oder ein- oder mehrfach ungesättigten Acylrest (-CO-R₁) mit insgesamt 1 bis 20, vorzugsweise 1 bis 7 Kohlenstoffatomen einschließlich seiner ein- oder mehrfach hydroxylierten Analoga; einen Aroylrest mit 6 bis 20, vorzugsweise 6 bis 10 Kohlenstoffatomen; einen Carbonylamidrest der Formel -CONH₂, -CONHR₁, -CONR₁R_{1'} oder -CONR₁R_{1'}R_{1"} ; einen linearen oder verzweigten, gesättigten oder ein- oder mehrfach ungesättigten Thioacylrest (-CS-R₁) mit insgesamt 1 bis 20, vorzugsweise 1 bis 7 Kohlenstoffatomen; oder einen Thiocarbamidrest der Formel -CS-NH₂, -CS-NHR₁, -CS-NR₁R_{1'} oder -CS-NR₁R_{1'}R_{1"};
wobei jeder der vorgenannten Reste außer H gegebenenfalls ein- oder mehrfach substituiert sein kann durch Halogen, insbesondere Fluor, Chlor, Brom oder Jod, Hydroxy-, Epoxy-, Amino-, Mercaptan-, Phenyl-, Phenol- oder Benzylgruppen, und
T einen Mono-, Di- oder Oligosaccharidrest mit bis zu 40 glykosidisch miteinander verknüpften, gegebenenfalls verzweigten Zuckerresten, die Furanose- und/oder Pyranoseringe darstellen und 5 bis 230 Kohlenstoffatome enthalten und N- oder O-glycosidisch an Polypeptide gebunden sind, und
Glycoproteine der allgemeinen Formel (I') für einen Glycosylphosphatidylinosit (GPI)-Anker 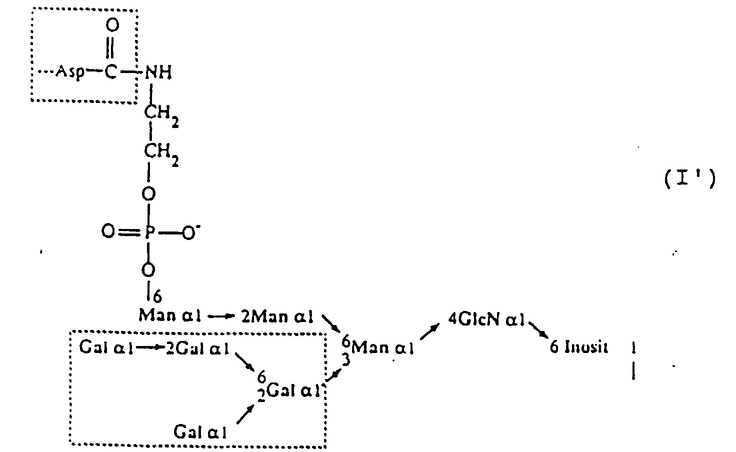 worin bedeuten:
Gal = Galactose,
Man = Mannose
Asp = Asparagin
GlcN = Glucosamin
wobei GlcN ersetzt sein kann durch ein Neuraminsäure-Vorstufen-Analogon der Formel (II) 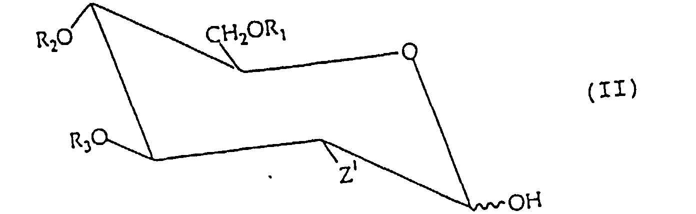 in der R₁, R₂ und R₃ die oben angegebenen Bedeutungen haben und Z' die gleichen Bedeutungen wie Z hat und neben der äquatorialen Position auch die axiale Position einnehmen kann und wobei außerdem bei äquatorialer Position von Z' der Rest -OR₂ in axialer Position stehen kann.

2. Verbindungen nach Anspruch 1, dadurch gekennzeichnet, daß in der allgemeinen Formel (I) T steht
für einen Saccharidrest mit N-Glycan-Struktur der Formel (Ia) 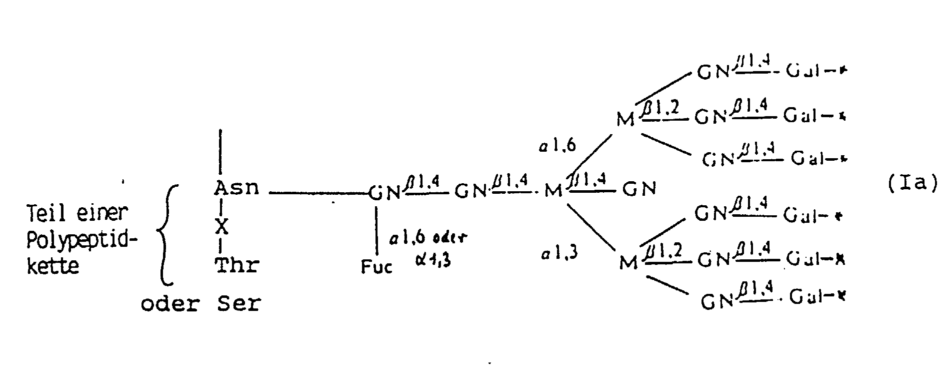 worin bedeuten:
Gal = Galactose,
GN = N-Acetyl-D-glucosamin,
M = D-Mannose,
Fuc = Fucose,
Asn = Asparagin,
X = eine beliebige Aminosäure außer Prolin,
Thr = Threonin,
Ser = Serin,
* = T-Verknüpfungsstelle (1 bis 6 Molekülreste),
wobei beide peripheren Reste M durch 1 bis 3 Trisaccharide substituiert sein können; oder
für einen Saccharidrest mit O-Glycan-Struktur der allgemeinen Formel (Ib): 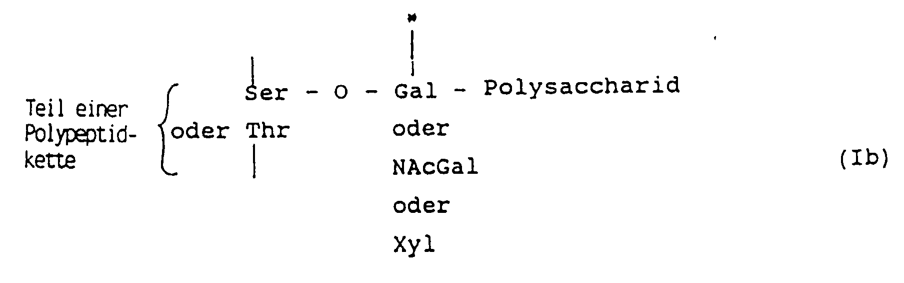 worin bedeuten:
Gal = Galactose
Thr = Threonin
Ser = Serin
Xyl = Xylose
NAcGal = N-Acetyl-galactosamin
* = T-Verknüpfungsstelle,
wobei in den obigen Formeln (Ia) und (Ib) die Galactose (Gal) ersetzt sein kann durch 2-Desoxy-galactose oder 2-Desoxy-2-halogenid(F, Cl, Br, J)-galactose.

3. Verbindungen nach Anspruch 1 und/oder 2, dadurch gekennzeichnet, daß dann, wenn T einen Saccharidrest mit N-Glycanstruktur oder einen Saccharidrest mit O-Glycanstruktur darstellt, GN steht für einen Rest der allgemeinen Formel (II): 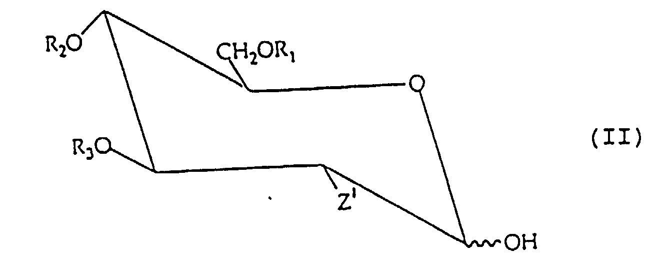 in der R₁, R₂ und R₃ die in Anspruch 1 angegebenen Bedeutungen haben und Z' die gleichen Bedeutungen wie Z hat und neben der äquatorialen Position auch die axiale Position einnehmen kann und wobei außerdem bei äquatorialer Position von Z' der Rest -OR₂ in axialer Position stehen kann.

4. Verbindungen nach mindestens einem der Ansprüche 1 bis 3, dadurch gekennzeichnet, daß R₂ bis R₅ stehen für H oder CH₃.

5. Aminozucker (Neuraminsäure-Vorstufen-Analoga) der allgemeinen Formel (II): 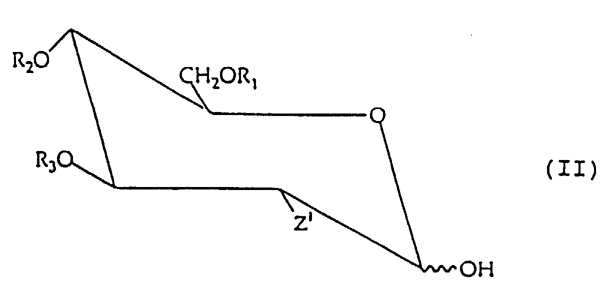 in der Z' und R₁, R₂ und R₃ die in den Ansprüchen 1 bis 4 angegebenen Bedeutungen haben.

6. Verfahren zur in vivo-Herstellung der Verbindungen nach den Ansprüchen 1 bis 5 durch parenterale oder enterale Verabreichung einer 2-Desoxy-2-amino-mannose, -glucose oder -galactose, deren Aminogruppe durch R₁ substituiert ist, vorzugsweise von N-Pentanoyl-, N-Hexanoyl-, N-Heptanoyl- oder N-Crotonoyl-D-mannosamin, an Mensch oder Tier.

7. Verfahren nach Anspruch 6, dadurch gekennzeichnet, daß die in vivo gebildeten Glycoproteine der Ansprüche 1 bis 4 für den therapeutischen Einsatz auf an sich bekannte Weise gewonnen (abgetrennt) werden.

8. Pharmazeutisches Mittel, dadurch gekennzeichnet, daß es als Wirkstoff, gegebenenfalls in Kombination mit anderen Wirkstoffen sowie üblichen pharmazeutischen Trägern und/oder Hilfsstoffen, enthält
mindestens ein Glycoprotein der Formel (I) 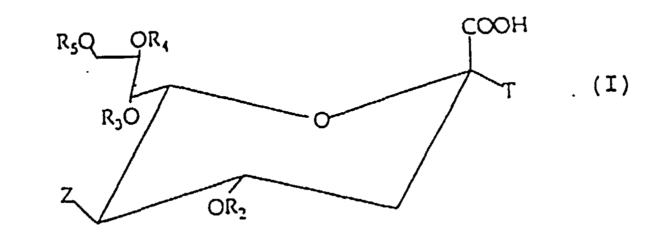 worin bedeuten:
Z -NHR, worin R den Pentanoyl-, Hexanoyl-, Heptanoyl- oder Crotonoylrest einschließlich seiner ein- oder mehrfach hydroxylierten Analoga darstellt
R₁, R_{1'} , R_{1"} , R₂, R₃, R₄ und R₅, die gleich oder verschieden sein können, jeweils Wasserstoff, einen linearen oder verzweigten Alkylrest mit 1 bis 20 Kohlenstoffatomen (CₙH₂ₙ₊₂, n = 1 bis 20), vorzugsweise 1 bis 7 Kohlenstoffatomen; einen linearen oder verzweigten Alkenylrest mit 3 bis 20 Kohlenstoffatomen (CₙH₂ₙ, n = 3 bis 20, Position der Doppelbindung an Cₙ n = 2 bis 19), vorzugsweise 3 bis 10 Kohlenstoffatomen; einen linearen oder verzweigten Alkinylrest mit 3 bis 20 Kohlenstoffatomen (CₙH₂ₙ₋₂, n = 3 bis 20, Position der Dreifachbindung an Cₙ n = 2 bis 19), vorzugsweise 3 bis 10 Kohlenstoffatomen; einen Alkenyl- bzw. Alkinylrest mit 2 oder mehr Doppelbindungen bzw. Dreifachbindungen mit 4 bis 20, vorzugsweise 7 bis 12 Kohlenstoffatomen; einen Arylrest mit 6 bis 20 Kohlenstoffatomen, vorzugsweise einen Phenylrest; einen linearen oder verzweigten, gesättigten oder ein- oder mehrfach ungesättigten Acylrest (-CO-R₁) mit insgesamt 1 bis 20, vorzugsweise 1 bis 7 Kohlenstoffatomen einschließlich seiner ein- oder mehrfach hydroxylierten Analoga; einen Aroylrest mit 6 bis 20, vorzugsweise 6 bis 10 Kohlenstoffatomen; einen Carbonylamidrest der Formel-CONH₂, -CONHR₁, -CONR₁R_{1'} oder -CONR₁R_{1'}R_{1"} ; einen linearen oder verzweigten, gesättigten oder ein- oder mehrfach ungesättigten Thioacylrest (-CS-R₁) mit insgesamt 1 bis 20, vorzugsweise 1 bis 7 Kohlenstoffatomen; oder einen Thiocarbamidrest der Formel -CS-NH₂, -CS-NHR₁, -CS-NR₁R_{1'} oder -CS-NR₁R_{1'}R_{1"};
wobei jeder der vorgenannten Reste außer H gegebenenfalls ein- oder mehrfach substituiert sein kann durch Halogen, insbesondere Fluor, Chlor, Brom oder Jod, Hydroxy-, Epoxy-, Amino-, Mercaptan-, Phenyl-, Phenol- oder Benzylgruppen, und
T einen Mono-, Di- oder Oligosaccharidrest mit bis zu 40 glykosidisch miteinander verknüpften, gegebenenfalls verzweigten Zuckerresten, die Furanose- und/oder Pyranoseringe darstellen und 5 bis 230 Kohlenstoffatome enthalten und N- oder O-glycosidisch an Polypeptide gebunden sind und/oder
mindestens ein Glycoprotein der allgemeinen Formel (I') für einen Glycosylphosphatidylinosit(GPI)-Anker 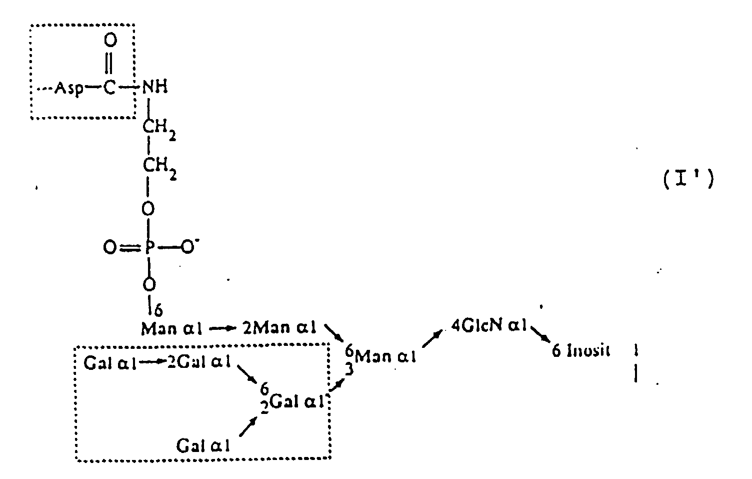 worin bedeuten:
Gal = Galactose,
Man = Mannose
Asp = Asparagin
GlcN = Glucosamin
wobei GlcN ersetzt sein kann durch ein Neuraminsäure-Vorstufen-Analogon der Formel (II) 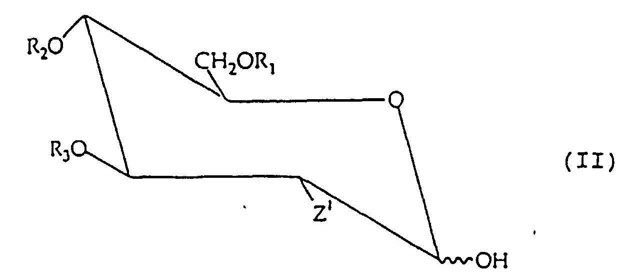 in der R₁, R₂ und R₃ die oben angegebenen Bedeutungen haben und Z' die gleichen Bedeutungen wie Z hat und neben der äquatorialen Position auch die axiale Position einnehmen kann und wobei außerdem bei äquatorialer Position von Z' der Rest -OR₂ in axialer Position stehen kann.

9. Pharmazeutisches Mittel nach Anspruch 8, dadurch gekennzeichnet, daß es den Wirkstoff in einer Menge von 0,01 bis 50 Gew.-%, vorzugsweise von 0,1 bis 20 Gew.-%, insbesondere von 2 bis 10 Gew.-%, enthält.

10. Pharmazeutisches Mittel nach Anspruch 8 oder 9 zur Stimulierung des Immunsystems, insbesondere der T-Lymphozyten, zur Infektabwehr, zur Behandlung von Immunschwächen, Tumorerkrankungen einschließlich Metastasierungsprozessen, Infektionskrankheiten (Viren, Bakterien, Parasiten, Protozoen) und Kreislaufversagen, insbesondere Gefäßverschlüssen und Septikämien bei Mensch und Tier.

11. Pharmazeutisches Mittel nach Anspruch 8 oder 9 zur Erhöhung der zytotoxischen Aktivität Natürlicher Killerzellen (NK-Zellen) zur Induktion einer Antitumor-Immunreaktion bei Mensch und Tier.

12. Pharmazeutisches Mittel nach Anspruch 8 oder 9 zur Steigerung der Phagozytose-Fähigkeit von Granulozyten und Monozyten zur Induktion einer Antitumor-Immunreaktion bei Mensch und Tier.

13. Pharmazeutisches Mittel nach Anspruch 8 oder 9 zur in vivo-Beeinflussung Neuraminsäure-abhängiger biologischer Prozesse.

14. Pharmazeutisches Mittel nach Anspruch 8 oder 9 zur Hemmung der Ligandenbindung an sialylierte Zelloberflächenrezeptoren (Endothelzellen, Thrombozyten, Leukozyten).

15. Pharmazeutisches Mittel nach Anspruch 8 oder 9 zur Hemmung der Bindung eines mikrobiellen Pathogens (Virus, Bakterium, Parasit, Protozoon) oder Toxins an die Wirtszelle über einen sialylierten Rezeptor durch in vivo-Modulation von Neuraminsäuren.

16. Pharmazeutisches Mittel nach Anspruch 8 oder 9 zur Stimulierung des Wachstums und der Differenzierung von menschlichen und tierischen Zellen des Immunsystems und zur Verhinderung der Adhäsion von Leukozyten, Thrombozyten und Tumorzellen an Gefäßendothelzellen.

17. Pharmazeutisches Mittel nach Anspruch 8 oder 9 zur biosynthetischen Herstellung von Liganden oder Rezeptoren mit modifizierter Neuraminsäure und deren Verwendung zur Kompetition physiologischer oder pathologischer Ligand-Rezeptor-Interaktionen.

18. Pharmazeutisches Mittel nach Anspruch 8 oder 9 zur in vitro-Beeinflussung des Infektionsverlaufs humaner Immundefizienz-Viren wie HIV-1 und HIV-2 sowie zur in vivo-Prävention der Infektion humaner Immundefizienz-Viren wie HIV-1 und HIV-2.

19. Pharmazeutisches Mittel nach Anspruch 8 oder 9 zur Behandlung von parasitären Erkrankungen, insbesondere von Trypanosomiasen, Leishmaniasen, Trichomoniasis, Giardiasis, Amöbiasis, Malaria, Pneumozystose, Schistosomiasis (Bilharziose) und Echinokokkose.

## Claims

1. Glycoproteins having the general formula (I) 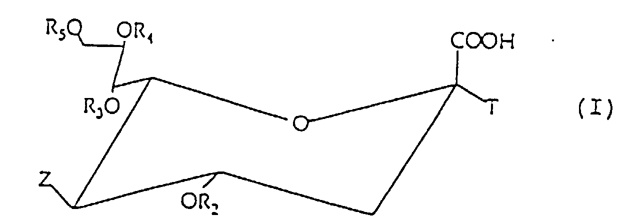 wherein
Z represents -NHR wherein R is a pentanoyl, hexanoyl, heptanoyl or crotonoyl residue or a mono- or multi-hydroxylated analogue of one of these residues,
R₁, R_{1'}, R_{1"}, R₂, R₃, R₄ and R₅ which can be the same or different each represent a hydrogen atom; a linear or branched alkyl residue containing 1 to 20 carbon atoms (CₙH₂ₙ₊₂, n = 1 to 20), preferably 1 to 7 carbon atoms; a linear or branched alkenyl residue containing 3 to 20 carbon atoms (CₙH₂ₙ, n = 3 to 20; double bond in Cₙ at C2 to C19 position), preferably 3 to 10 carbon atoms; a linear or branched alkynyl residue containing 3 to 20 carbon atoms (CₙH₂ₙ₋₂, n = 3 to 20; triple bond in Cₙ at C2 to C19 position), preferable 3 to 10 carbon atoms; an alkenyl or alkynyl residue having 2 or more double and triple bonds respectively, and containing 4 to 20, preferably 7 to 12 carbon atoms; an aryl residue containing 6 to 20 carbon atoms, preferably a phenyl residue; a linear or branched, saturated or mono- or multi-unsaturated acyl residue (-CO-R₁), containing a total of 1 to 20, preferably 1 to 7 carbon atoms, including its mono- or multi-hydroxylated analogues; an aroyl residue containing 6 to 20, preferably 6 to 10 carbon atoms; a carbonylamide residue of the formula -CONH₂, -CONHR₁, -CONR₁R_{1'} or -CONR₁R_{1'}R_{1"} ; a linear or branched, saturated or mono- or multi-unsaturated thioacyl residue (-CS-R₁), containing a total of 1 to 20, preferably 1 to 7 carbon atoms; or a thiocarbamide residue of the formula -CS-NH₂, -CS-NHR₁, -CS-NR₁ R_{1'} or -CS-NR₁R_{1'}R_{1"};
wherein each of the aforementioned residues, with the exception of H, optionally can be singly or multiply substituted by halogen, preferably fluorine, chlorine, bromine or iodine, hydroxy, epoxy, amino, mercaptane, phenyl, phenol or benzyl groups; and
T represents a mono-, di- or oligosaccharide residue with up to 40 glycosidically linked, optionally or branched sugar residues representing furanose and/or pyranose rings, linked N- or O-glycosidically to the polypeptide and containing 5 to 230 carbon atoms; and
glycoproteins having the general formula (I'), which serve as a glycosylphosphatidylinositol(GPI) anchor: 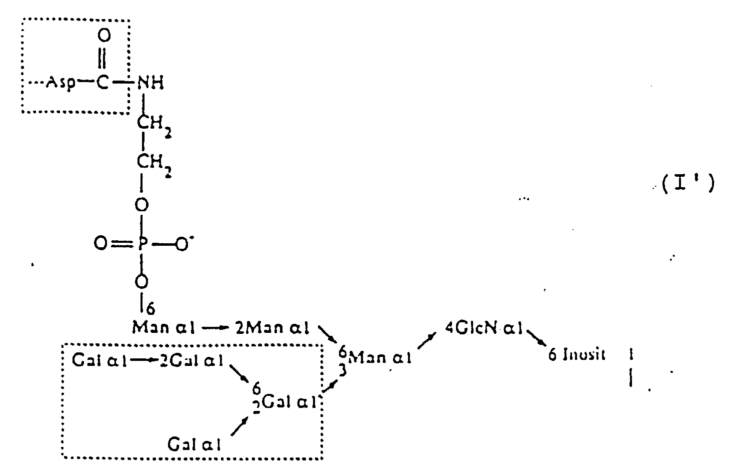 where
Gal = galactose,
Man = mannose,
Asp = asparagine,
GlcN = glucosamine,
and in which GlcN can be replaced by an analogue of a neuraminic acid precursor having the formula (II) 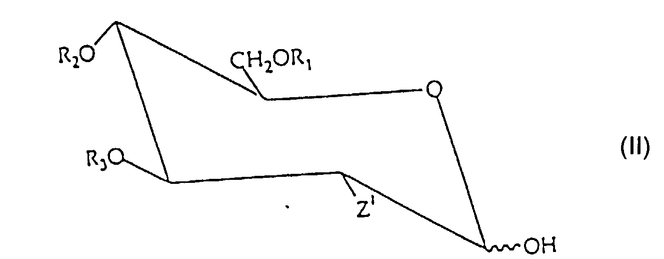 wherein R₁, R₂ and R₃ have the same meaning as above and Z' is the same as Z and can occupy an equatorial position as well as an axial position and wherein furthermore, if Z' occupies the equatorial position, the axial position can be occupied by -OR₂.

2. The compounds according to claim 1, characterized in that T in general formula (I) represents either a saccharide residue having an N-glycan structure having the formula (la) 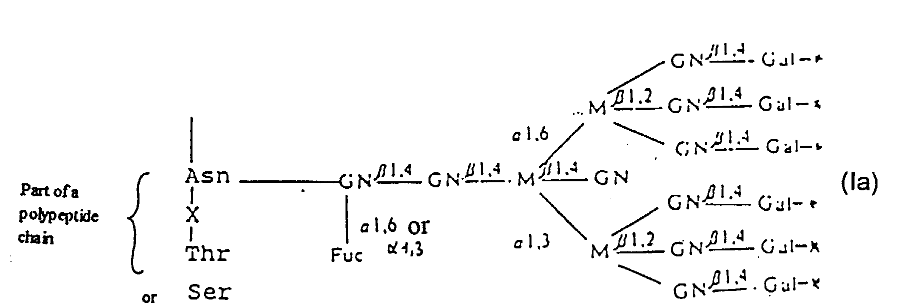 where
Gal = galactose,
GN = N-acetyl-D-glucosamine,
M = D-mannose,
Fuc = fucose,
Asn = asparagine,
X = any amino acid except proline,
Thr = threonine,
Ser = serine,
* = attachment site of T (1 to 6 molecular residues),
in which both peripheral M residues can be substituted by 1 to 3 trisaccharides; or
a saccharide residue having an 0-glycan structure having the general formula (Ib): 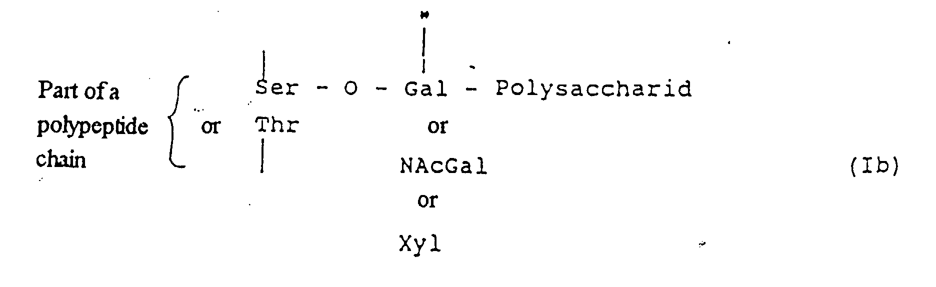 where
Gal = galactose
Thr = threonine
Ser = serine
Xyl = xylose
NAcGal = N-acetylgalactosamine
* = attachment site of T,
and wherein in the above formulae (la) and (Ib), galactose (Gal) can be replaced by 2-deoxy-galactose or 2-deoxy-2-halide(F, Cl, Br, I)-galactose.

3. The compounds according to claim 1 and/or 2, characterized in that when T is a saccharide residue having an N-glycan or an O-glycan structure, GN is a residue of the general formula (II): 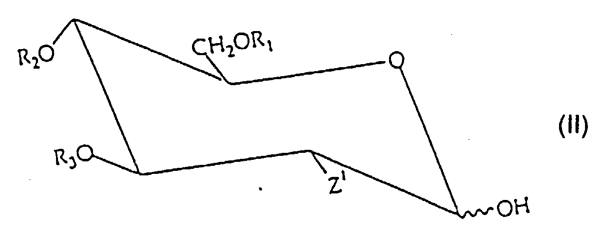 wherein R₁, R₂ and R₃ have the meaning as defined in claim 1; and Z' is the same as Z and can occupy an equatorial position as well as an axial position; and wherein furthermore, if Z' occupies the equatorial position, the axial position can be accupied by -OR₂.

4. The compounds according to at least one of claims 1 to 3, characterized in that R₂ to R₅ represent H or CH₃.

5. Aminosugars (analogues of neuraminic acid precursors) having the general formula (II): 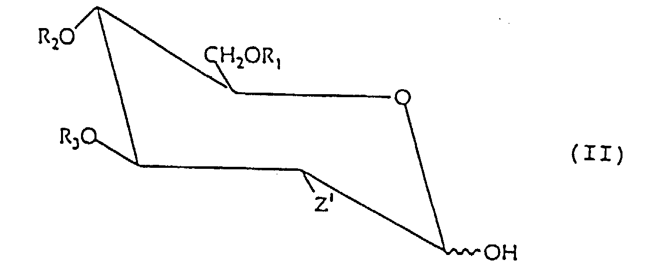 wherein Z' and R₁, R₂ and R₃ have the same meaning as defined in claims 1 to 4.

6. A method for the in vivo-preparation of the compounds according to claims 1 to 5 by parenteral or enteral administration to humans or animals of a 2-deoxy-2-amino-mannose, -glucose or -galactose, wherein the amino group is substituted by R₁ ; preferably of N-pentanoyl-, N-hexanoyl-, N-heptanoyl- or N-crotonoyl-D-mannosamine.

7. The method according to claim 6, characterized in that the in vivo-synthesized glycoproteins of claims 1 to 4 are obtained(separated) in a manner known per se and are used therapeutically.

8. A pharmaceutical preparation characterized in that it contains as an active constituent, optionally in combination with other active constituents, usual pharmaceutical vehicles and/or auxiliary substances, at least one glycoprotein having the formula (I) 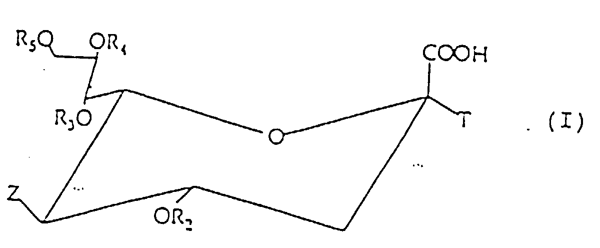 wherein
Z represents -NHR wherein R is a pentanoyl, hexanoyl, heptanoyl or crotonoyl residue or a mono- or multi-hydroxylated analogue of one of these residues,
R₁, R_{1'}, R_{1"}, R₂, R₃, R₄ and R₅ which can be the same or different each represent a hydrogen atom; a linear or branched alkyl residue containing 1 to 20 carbon atoms (CₙH₂ₙ₊₂, n = 1 to 20), preferably 1 to 7 carbon atoms; a linear or branched alkenyl residue containing 3 to 20 carbon atoms (CₙH₂ₙ, n = 3 to 20; double bond in Cₙ at C2 to C19 position), preferably 3 to 10 carbon atoms; a linear or branched alkynyl residue containing 3 to 20 carbon atoms (CₙH₂ₙ₋₂, n = 3 to 20; triple bond in Cₙ at C2 to C19 position), preferable 3 to 10 carbon atoms; an alkenyl or alkynyl residue having 2 or more double and triple bonds respectively, and containing 4 to 20, preferably 7 to 12 carbon atoms; an aryl residue containing 6 to 20 carbon atoms, preferably a phenyl residue; a linear or branched, saturated or mono- or multi-unsaturated acyl residue (-CO-R₁), containing a total of 1 to 20, preferably 1 to 7 carbon atoms, including its mono- or multi-hydroxylated analogues; an aroyl residue containing 6 to 20, preferably 6 to 10 carbon atoms; a carbonylamide residue of the formula -CONH₂, -CONHR₁, -CONR₁R_{1'} or -CONR₁R_{1'}R_{1"}; a linear or branched, saturated or mono- or multi-unsaturated thioacyl residue (-CS-R₁), containing a total of 1 to 20, preferably 1 to 7 carbon atoms; or a thiocarbamide residue of the formula -CS-NH₂, -CS-NHR₁, -CS-NR₁R_{1'} or -CS-NR₁R_{1'}R_{1"}; wherein each of the aforementioned residues, with the exception of H, optionally can be singly or multiply substituted by halogen, preferably fluorine, chlorine, bromine or iodine, hydroxy, epoxy, amino, mercaptane, phenyl, phenol or benzyl groups; and
T represents a mono-, di- or oligosaccharide residue with up to 40 glycosidically linked, optionally branched sugar residues representing furanose and/or pyranose rings, linked N- or O-glycosidically to the polypeptide and containing 5 to 230 carbon atoms; and/or
at least one glycoprotein having the general formula (I'), which serve as a glycosylphosphatidylinositol(GPI) anchor: 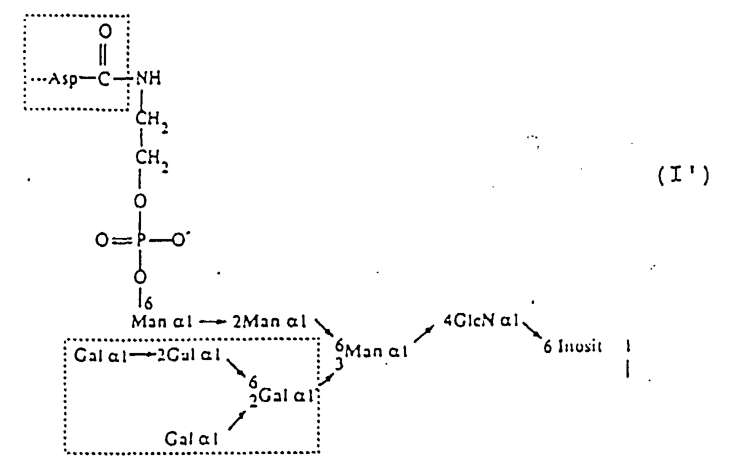 where
Gal = galactose,
Man = mannose,
Asp = asparagine,
GlcN = glucosamine,
and in which GlcN can be replaced by an analogue of a neuraminic acid precursor having the formula (II) 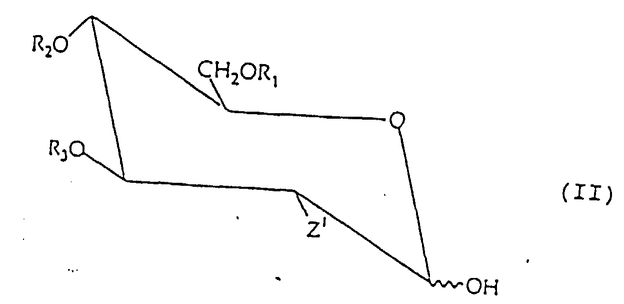 wherein R₁, R₂ and R₃ have the same meaning as above and Z' is the same as Z and can occupy an equatorial position as well as an axial position and wherein furthermore, if Z' occupies the equatorial position, the axial position can be occupied by -OR₂.

9. The pharmaceutical preparation according to claim 8, characterized in that it contains the active constituent in an amount of 0.1 to 50% by weight, preferably 0.1 to 20 % by weight, especially 2 to 10% by weight.

10. The pharmaceutical preparation according to claim 8 or 9 for stimulation of the immune system, in particular of the T-lymphocytes, for the protection against infection, for the treatment of a weak immune response, of tumor diseases including processes of metastasis, of infectious diseases (caused by viruses, bacteria, parasites, protozoa) and of circulatory collapse, in particular vascular obliterations and septicaemia, in humans and animals.

11. The pharmaceutical preparation according to claim 8 or 9 for increasing the cytotoxic activity of natural killer cells (NK cells) for inducing an antitumor immune-reaction in humans and animals.

12. The pharmaceutical preparation according to claim 8 or 9 for increasing the phagocytic activity of granulocytes and monocytes for the induction of an anti-tumor immune-reaction in humans and animals

13. The pharmaceutical preparation according to claim 8 or 9 for the in vivo-modulation of neuraminic acid-dependent processes.

14. The pharmaceutical preparation according to claim 8 or 9 for the inhibition of the ligand binding to sialylated cell surface receptors (endothelial cells, thrombocytes, leucozytes).

15. The pharmaceutical preparation according to claim 8 or 9 for the inhibition of the binding of a pathogenic microorganism (virus, bacterium, parasite, protozoan) or of a toxin to a host cell via a sialylated receptor by in vivo-modulation of neuraminic acids.

16. The pharmaceutical preparation according to claim 8 or 9 for stimulation of the growth and differentiation of human and animal cells of the immune system, and for preventing adhesion of leucozytes, thrombocytes and tumor cells to vascular endothel cells.

17. The pharmaceutical preparation according to claim 8 or 9 for the biosynthetic preparation of ligands or receptors with modified neuraminic acid, and its use as physiological or pathological competitors of ligand-receptor interactions.

18. The pharmaceutical preparation according to claim 8 or 9 for the in vitro-modulation of the course of infection by human immune deficiency viruses e.g. HIV-1 and HIV-2 as well as for the in vivo prevention of infection by human immune deficiency viruses e.g. HIV-1 and HIV-2.

19. The pharmaceutical preparation according to claim 8 or 9 for the treatment of parasitic diseases, in particular trypanosomiasis, leishmaniasis, trichomoniasis, giardiasis, amoebiasis, malaria, pneumocystosis, schistosomiasis (Bilharzia) and echinococcosis.

## Revendications

1. Glycoprotéines de la formule générale 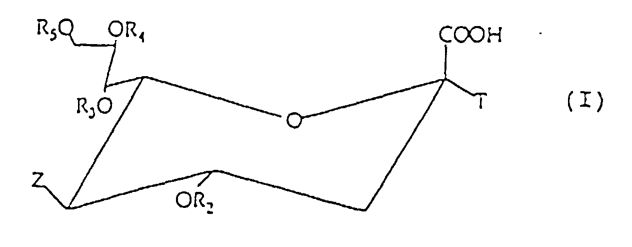 dans laquelle
Z signifie -NHR, R représentant le reste pentanoyle, hexanoyle, heptanoyle ou crotonoyle, y inclus ses analogues mono- ou multi-hydroxylés,
R₁, R_{1'}, R_{1"}, R₂, R₃, R₄ et R₅, qui peuvent être identiques ou différents, représentent respectivement l'hydrogène, un reste alkyle linéaire ou ramifié avec 1 à 20 atomes de carbone (CₙH₂ₙ₊₂, n = 1 à 20), de préférence 1 à 7 atomes de carbone; un reste alkylène linéaire ou ramifié avec 3 à 20 atomes de carbone (CₙH₂ₙ, n = 3 à 20, position de la double liaison dans Cₙ n = 2 à 19), de préférence 3 à 10 atomes de carbone; un reste alkinyle linéaire ou ramifié avec 3 à 20 atomes de carbone (CₙH₂ₙ₋₂, n = 3 à 20, position de la triple liaison dans Cₙ n = 2 à 19), de préférence 3 à 10 atomes de carbone; un reste alkényle respectivement alkinyle avec 2 ou plus doubles ou triples liaisons avec 4 à 20, de préférence 7 à 12 atomes de carbone; un reste aryle avec 6 à 20 atomes de carbone, de préférence un reste phényle; un reste acyle (-CO-R₁) linéaire ou ramifié, saturé ou mono- ou multi-insaturé avec en tout 1 à 20, de préférence 1 à 7 atomes de carbone, y inclus ses analogues mono- ou multi-hydroxylés; un reste aroyle avec 6 à 20, de préférence 6 à 10 atomes de carbone; un reste carbonylamide de la formule -CONH₂, -CONHR₁, -CONR₁R_{1'}, ou -CONR₁R_{1'}R_{1"}; un reste thioacyle (-CS-R₁) linéaire ou ramifié, saturé ou mono- ou multi-insaturé avec en tout 1 à 20, de préférence 1 à 7 atomes de carbone; ou un reste thiocarbamide de la formule -CS-NH₂, -CS-NHR₁, -CS-NR₁R_{1'} ou -CS-NR₁R_{1'}R_{1"}; chacun desdits restes, à l'exception de H, pouvant éventuellement être mono- ou multi-substitué par un halogène, en particulier le fluor, le chlore, le brome ou le iode, des groupes hydroxy, époxy, amino, mercaptane, phényle, phénol ou benzyle, et
T représente un reste d'un mono-, di- ou oligosaccharide avec jusqu'à 40 restes de sucre entreliés glycosidiquement, éventuellement ramifiés, qui représentent des anneaux de furanose et/ou de pyranose et qui contiennent 5 à 230 atomes de carbone et sont liés à des polypeptides de façon N- ou O-glycosidique, et
des glycoprotéines de la formule générale (I') pour un ancre de glycosylphosphatidylinosite (GPI) 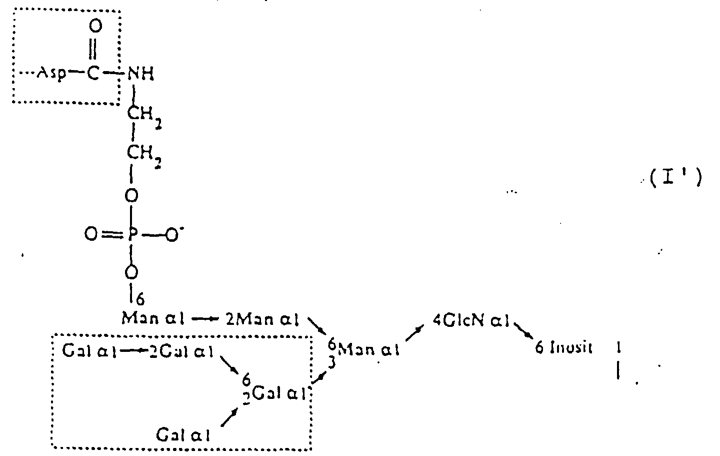 dans laquelle signifient:
Gal = galactose,
Man = mannose,
Asp = asparagine,
GlcN = glucosamine
le GlcN pouvant être remplacé par un analogue précurseur de l'acide neuraminique de la formule (II) 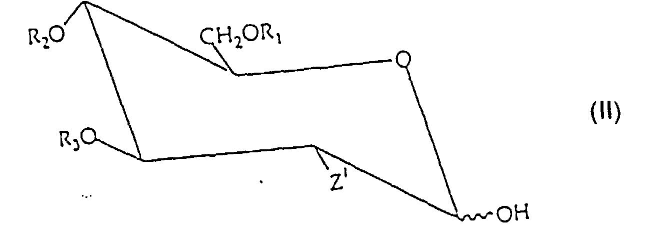 dans laquelle R₁, R₂ et R₃ ont les significations indiqués ci-dessus et Z' a la même signification que Z et peut prendre la position axiale aussi bien que la position équatoriale, et dans laquelle, si Z' se trouve dans la position équatoriale, le reste -OR₂ peut occuper la position axiale.

2. Composés selon la revendication 1, caractérisés par le fait que dans la formule générale (I) T représente
un reste d'un saccharide avec une structure de N-glycane de la formule (Ia) 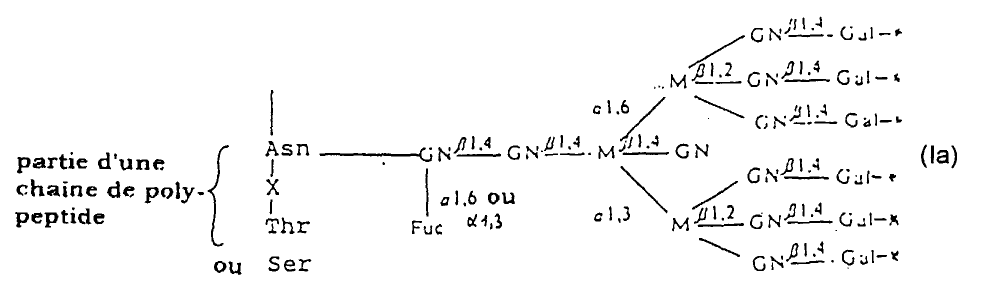 dans laquelle signifient:
Gal = galactose,
GN = N-acétyle-D-glucosamine,
M = D-mannose,
fuc = fucose,
Asn = asparagine,
X = un acide aminé à l'exception de proline,
Thr = threonine,
Ser = sérine
* = endroit de T-enchaînement (1 à 6 restes de molécule)
les deux restes périphériques M pouvant être substitués par 1 à 3 trisaccharides; ou
un reste d'un saccharide avec une structure d'O-glycane de la formule générale (Ib) 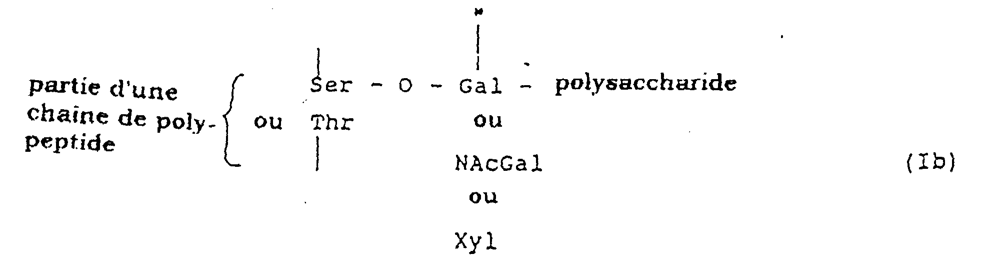 dans laquelle signifient:
Gal = galactose
Thr = thréonine
Ser = sérine
Xyl = xylose
NAcGal = N-acétyle-galactosamine
* = endroit de T-enchaînement
dans les formules (Ia) et (Ib) la galactose (Gal) pouvant être remplacée par la 2-desoxy-galactose ou la 2-desoxy-2-halogénide (F, Cl, Br, I)-galactose.

3. Composés selon la revendication 1 et/ou 2, caractérisés par le fait que, lorsque T représente un reste d'un saccharide avec une structure de N-glycane ou un reste d'un saccharide avec une structure de O-glycane, GN représente un reste de la formule générale (II): 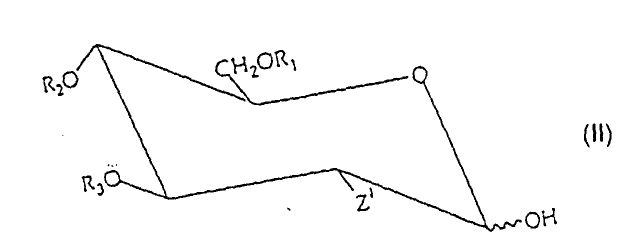 dans laquelle R₁, R₂ et R₃ ont les significations indiquées dans la revendication 1 et Z' a les mêmes significations que Z et peut occuper aussi bien la position axiale que la position équatoriale et dans laquelle, en outre, si Z' se trouve dans la position équatoriale, le reste -OR₂ peut occuper la position axiale.

4. Composés selon au moins l'une des revendications 1 à 3, caractérisés par le fait que R₂ à R₅ représentent H ou CH₃.

5. Sucres aminés (analogues précurseurs de l'acide neuraminique) de la formule générale (II): 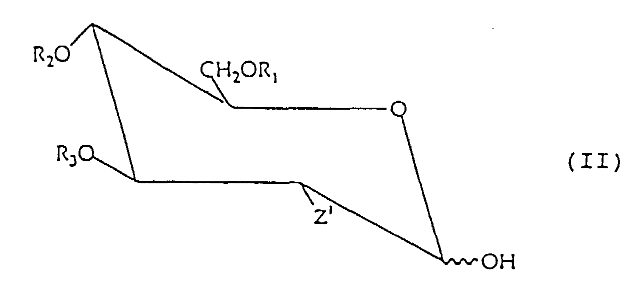 dans laquelle Z' et R₁, R₂ et R₃ ont les significations indiquées dans les revendications 1 à 4.

6. Procédé pour la formation in-vivo des composés selon les revendications 1 à 5 par administration parentérale ou entérale aux êtres humains ou aux animaux d'une 2-desoxy-2-amino-mannose, -glucose ou -galactose, dont le groupe amino est substitué par R₁, de préférence par N-pentanoyle, N-hexanoyle, N-heptanoyle ou N-crotonoyle-d-mannosamine.

7. Procédé selon la revendication 6, caractérisé par le fait que les glycoprotéines formés in-vivo selon les revendications 1 à 4 sont obtenus (séparés) de façon en soi connue pour l'utilisation thérapeutique.

8. Produit pharmaceutique, caractérisé par le fait qu'il contient en tant que substance active, le cas échéant en combinaison avec d'autres substances actives ainsi que des véhicules pharmaceutiques classiques et/ou des substances auxiliaires:
au moins un glycoprotéine de la formule (I) 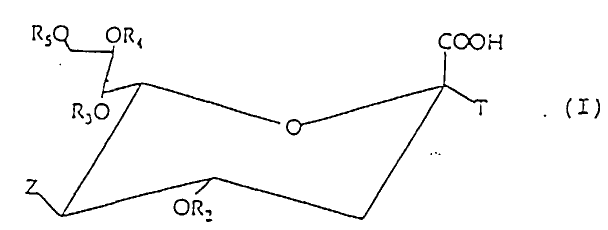 dans laquelle
Z signifie -NHR, R représentant le reste pentanoyle, hexanoyle, heptanoyle ou crotonoyle, y inclus ses analogues mono- ou multi-hydroxylés,
R₁, R_{1'}, R_{1"}, R₂, R₃, R₄ et R₅, qui peuvent être identiques ou différents, représentent respectivement l'hydrogène, un reste alkyle linéaire ou ramifié avec 1 à 20 atomes de carbone (CₙH₂ₙ₊₂, n = 1 à 20), de préférence 1 à 7 atomes de carbone; un reste alkylène linéaire ou ramifié avec 3 à 20 atomes de carbone (CₙH₂ₙ, n = 3 à 20, position de la double liaison dans Cₙ n = 2 à 19), de préférence 3 à 10 atomes de carbone; un reste alkinyle linéaire ou ramifié avec 3 à 20 atomes de carbone (CₙH₂ₙ₋₂, n = 3 à 20, position de la triple liaison dans Cₙ n = 2 à 19), de préférence 3 à 10 atomes de carbone; un reste alkényle respectivement alkinyle avec 2 ou plus doubles ou triples liaisons avec 4 à 20, de préférence 7 à 12 atomes de carbone; un reste aryle avec 6 à 20 atomes de carbone, de préférence un reste phényle; un reste acyle (-CO-R₁) linéaire ou ramifié, saturé ou mono- ou multi-insaturé avec en tout 1 à 20, de préférence 1 à 7 atomes de carbone, y inclus ses analogues mono- ou multi-hydroxylés; un reste aroyle avec 6 à 20, de préférence 6 à 10 atomes de carbone; un reste carbonylamide de la formule -CONH₂, -CONHR₁, -CONR₁R_{1'}, ou -CONR₁R_{1'}R_{1"}; un reste thioacyle (-CS-R₁) linéaire ou ramifié, saturé ou mono- ou multi-insaturé avec en tout 1 à 20, de préférence 1 à 7 atomes de carbone; ou un reste thiocarbamide de la formule -CS-NH₂, -CS-NHR₁, -CS-NR₁R_{1'} ou -CS-NR₁R_{1'}R_{1"}; chacun desdits restes à l'exception de H pouvant, le cas échéant, être mono- ou multi-substitué par un halogène, en particulier le fluor, le chlore, le brome ou le iode, des groupes hydroxy, époxy, amino, mercaptane, phényle, phénol ou benzyle, et
T représente un reste d'un mono-, di- ou oligosaccharide avec jusqu'à 40 restes de sucre entreliés glycosidiquement, éventuellement ramifiés, qui représentent des anneaux de furanose et/ou de pyranose et qui contiennent 5 à 230 atomes de carbone et sont liés à des polypeptides de façon N- ou O-glycosidique, et/ou
au moins un glycoprotéine de la formule générale (I') pour un ancre de glycosylphosphatidylinosite (GPI) 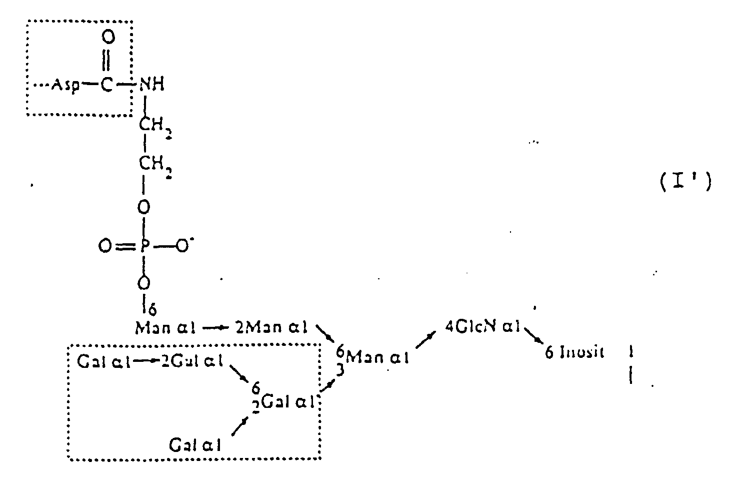 dans laquelle signifient:
Gal = galactose,
Man = mannose,
Asp = asparagine,
GlcN = glucosamine
le GlcN pouvant être remplacé par un analogue précurseur de l'acide neuraminique de la formule (II) 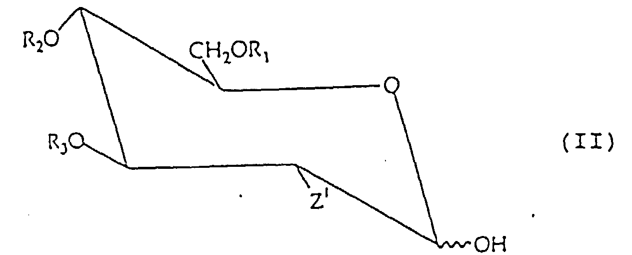 dans laquelle R₁, R₂ et R₃ ont les significations indiqués ci-dessus et Z' a les mêmes significations que Z et peut prendre la position axiale aussi bien que la position équatoriale, et dans laquelle en outre, si Z' se trouve dans la position équatoriale, le reste -OR₂ peut occuper la position axiale.

9. Produit pharmaceutique selon la revendication 8, caractérisé par le fait qu'il contient la substance active dans une quantité comprise entre 0,01 et 50% en poids, de préférence entre 0,1 et 20% en poids, en particulier entre 2 et 10% en poids.

10. Produit pharmaceutique selon la revendication 8 ou 9 pour la stimulation du système immunitaire, en particulier des T-lymphocytes, pour la protection contre les infections, pour le traitement des déficiences immunitaires, des maladies tumeurs, y inclus des processus de formation de métastases, des maladies infectieuses (virus, bactéries, parasites, protozoes) et collapsus circulatoire, en particulier des oblitérations vasculaires et septicémies auprès de l'être humain et de l'animal.

11. Produit pharmaceutique selon la revendication 8 ou 9 pour l'augmentation de l'activité cytotoxique des cellules "killer" naturelles (cellules NK) pour l'induction d'une réaction immunitaire anti-tumeur auprès de l'être humain et de l'animal.

12. Produit pharmaceutique selon la revendication 8 ou 9 pour l'augmentation de l'aptitude de phagocytose des granulocytes et des monocytes pour l'induction d'une réaction immunitaire anti-tumeur auprès de l'être humain et de l'animal.

13. Produit pharmaceutique selon la revendication 8 ou 9 pour influencer in-vivo des processus biologiques dépendant de l'acide neuraminique.

14. Produit pharmaceutique selon la revendication 8 ou 9 pour l'inhibition de la liaison de ligands à des récepteurs de surface de cellule sialylés (cellules endothéliales, thrombocytes, leucocytes).

15. Produit pharmaceutique selon la revendication 8 ou 9 pour l'inhibition de la liaison d'un micro-organisme pathogène (virus, bactérie, parasite, protozoon) ou d'un toxine à la cellule hôte par l'intermédiaire d'un récepteur sialylé par modulation in-vivo des acides neuraminiques.

16. Produit pharmaceutique selon la revendication 8 ou 9 pour la stimulation de la croissance et de la différentiation de cellules humaines ou animales du système immunitaire et pour empêcher l'adhésion des leucocytes, des thrombocytes et des cellules de tumeurs à des cellules vasculaires endothéliales.

17. Produit pharmaceutique selon la revendication 8 ou 9 pour la formation biosynthétique de ligands ou de récepteurs avec un acide neuraminique modifié et leur utilisation pour la compétition d'interactions ligand-récepteur physiologiques ou pathologiques.

18. Produit pharmaceutique selon la revendication 8 ou 9 pour influencer in-vitro la course de l'infection des virus de déficience immunitaire humaine comme HIV-1 et HIV-2 ainsi que pour la prévention in-vivo de l'infection avec des virus de déficience immunitaire humaine comme HIV-1 et HIV-2.

19. Produit pharmaceutique selon la revendication 8 ou 9 pour le traitement de maladies parasitaires, en particulier de trypanosomiases, leishmaniases, trichomoniasis, giardiasis, amoebiasis, malaria, pneumocystosis, schistosomiasis (bilharziosis) et échinococcosis.
